# EUROPEAN PATENT SPECIFICATION

(11) **EP 3 773 730 B1**
(45) Date of publication and mention of the grant of the patent: **13.05.2026**
(21) Application number: 19776288.3
(22) Date of filing: 26.03.2019
(51) Int. Cl.: A61K 47/02, A61K 9/00, A61K 9/14, A61K 9/16, A61K 47/00, A61K 9/51, A61K 31/567, A61K 31/57, A61K 47/28

(54) **DRUG DELIVERY FORMULATIONS**
WIRKSTOFFFREISETZUNGSFORMULIERUNGEN
FORMULATIONS D'ADMINISTRATION DE MÉDICAMENT

(30) Priority: 27.03.2018 US 201862648905 P
(43) Date of publication of application: 17.02.2021
(73) Proprietor: The Regents of the University of California, Oakland, CA 94607-5200 (US)
(72) Inventor: SAILOR, Michael J., La Jolla, CA 92037 (US); HOLLETT, Geoffrey Ian, Sacramento, CA 95811 (US); INGALLINERA, Thomas, Hunt Valley, MD 21031 (US)
(74) Representative: Gill Jennings & Every LLP
(86) International application number: PCT/US2019/024131
(87) International publication number: WO 2019/191132

(56) References cited:
- EP-A1- 2 870 960
- WO-A1-2015/153613
- WO-A2-2007/071915
- US-A1- 2003 031 721
- US-A1- 2007 071 787
- US-A1- 2015 011 521
- HELDER A. SANTOS ET AL: "Multifunctional Porous Silicon for Therapeutic Drug Delivery and Imaging", CURRENT DRUG DISCOVERY TECHNOLOGIES, vol. 8, no. 3, 1 September 2011 (2011-09-01), US, pages 228 - 249, XP055636588, ISSN: 1570-1638, DOI: 10.2174/157016311796799053

## Description

### TECHNICAL FIELD

The disclosure provides drug delivery formulations that comprise a porous silicon material loaded with a meltable compound or composition. The meltable compositions comprise a melting point suppression agent. The disclosure further provides methods of making said drug delivery formulations and uses thereof.

### BACKGROUND

Formulations suitable for the controlled delivery of active therapeutic agents over long periods of time continue to be a subject of intense interest and effort in the pharmaceutical and therapeutic sciences. In particular, the long-term delivery of therapeutic agents having low solubility in aqueous solutions can be especially difficult.

One area where the development of formulations to provide long-term delivery of a therapeutic agent is of particular importance is in the delivery of birth control agents. Access to reliable and safe contraception is a critical component to lowering maternal death rates while simultaneously granting women agency over their lives. Effective family planning tools have been linked to positive health outcomes, but they also have the societal benefit of increasing women's participation in the workforce and enrollment in professional and graduate level training. Currently, however, there are large populations of women who wish to use contraception but ultimately do not. According to the World Health Organization's (WHO) 2015 report on Trends in Contraceptive Use Worldwide, 12% of married or in-union women between the ages of 15 and 49 had unmet contraceptive needs. In addition, it is estimated that over 500,000 women die each year from pregnancy-related complications.

Subcutaneous or intramuscular injection of a long-acting drug formulation is a large and growing approach to contraception - it is the form most widely used in sub-Saharan Africa, at more than double the rate of its next highest competitor, the daily oral pill (10.7% vs 5.1%). By far, the most popular injection is depot medroxyprogesterone acetate (DMPA, Depo-Provera^{®}), with over 30 million doses procured by the United Nations Population Fund in 2015. Since its release in 1960, there have been minimal innovations in the field of injectable contraceptives despite the obvious drawbacks of DMPA. In the United States, the Food and Drug Administration has stipulated a "black box" warning label on DMPA, noting that women who use DMPA may experience significant bone mineral density loss over time. Oral formulations of MPA (Provera^{®}) do not carry the same warning label, and some doctors have expressed concern that the label limits access to an important tool for women's health.

There have been few innovations in the field of injectable contraceptives apart from modifications in drug (norethisterone enanthate, NET-EN), or packaging (SayanaPress^{®}), and the field has relied on the same depot injection of a pure crystalline drug for more than 50 years. By contrast, diseases such as cancer, bipolar disorder, and type 2 diabetes have all seen improved patient outcomes by switching from crystalline drug injections to host material formulations that can better control the pharmacokinetic profile of the drug. Given the number of women who use injectable contraception and its impact on maternal morbidity and mortality, it is surprising that there has been little improvement on DMPA. Extended release profiles for contraceptive drugs have been successfully observed in implants such as Norplant^{®}, but they require doctor supervision for implantation and removal and cannot be self-administered by the patient.

The two most important issues with DMPA injections relate to the pharmacokinetic profile: there is an initial excessive burst of MPA in the serum, and MPA concentration drops very slowly at the end of the intended dose period. To ensure sufficiently long action of the contraceptive, the injected dose is quite large, and the high MPA concentration during the weeks following injection (the burst release phase) is largely responsible for bone mineral density loss. The long tail is an issue for many women timing their return to fertility. One study found that the median return to fertility for DMPA users was approximately 9 months post-injection, which is nearly three times longer than the intended 3-month coverage indicated for DMPA. In extreme cases, ovulation did not return for more than 10 months after DMPA was supposed to have cleared from the body.

Traditional approaches for extending drug release utilize polymer-based materials, such as poly(lactic-co-glycolic) acid (PLGA). This strategy has seen success in a variety of treatments with products such as Risperdal Consta^{®} (bipolar disorder), Sandostatin LAR^{®} (carcinoid syndrome), and Bydureon^{®} (type 2 diabetes). By incorporating the drug into a host material, the surface area of exposed drug is minimized and the drug is only released when the polymer dissolves. While microsphere hosts provide longer duration of drug delivery, their release profiles show "burst-and-tail" shapes similar to the crystalline drug. Polymer-based materials have the additional drawbacks of requiring a cold chain for distribution and of showing susceptibility to heat- or gamma irradiation-based terminal sterilization methods that negatively impact pharmacokinetic behavior.

### SUMMARY

This disclosure solves these and other problems by providing innovative drug delivery formulations for the administration of therapeutic agents based on biodegradable porous silicon materials. Demonstrated herein is the mass loading of porous silicon materials with, for example, contraceptive agents and antibiotics using a melt casting approach. It is further shown that these drug-loaded porous silicon materials exhibit a temporal and linear release profile of an optimized formulation for an extended period of time (90 days), with an overall estimated release duration *in vivo* of greater than 6 months. Moreover, the release profile showed a rapid taper at the end of the release. Thus, the performance of the drug delivery formulations is superior to "free" segesterone acetate. The drug delivery formulations have reproducible syringability and exhibit a low toxicity *in vivo* in rats.

The present invention provides a drug delivery formulation comprising: a porous silicon material loaded with a meltable composition, wherein the meltable composition comprises a therapeutic agent and a melting point suppression agent, wherein the therapeutic agent is a contraceptive drug selected from the group consisting of segesterone, etonogestrel, levonorgestrel, levonorgestrel butanoate, and medroxyprogesterone acetate, and wherein the melting point suppression agent is a steroid or a polyketide, wherein the meltable composition has a melting temperature, wherein the therapeutic agent has a melting temperature and a decomposition temperature, and wherein the melting temperature of the meltable composition is lower than the melting temperature of the therapeutic agent and the decomposition temperature of the therapeutic agent.

The disclosure provides for a porous silicon material having a porosity from about 15% to about 85%, wherein the pores of the silicon material are loaded with a mixture comprising a thermally unstable therapeutic agent and a thermally unstable substance, wherein the thermally unstable therapeutic agent and the thermally unstable substance are not the same compound or molecule, and wherein the mixture has a lower melting point than the melting point of the thermally unstable therapeutic agent. In another embodiment, the porous silicon material disclosed herein are porous silicon particles. In yet another embodiment, the porous silicon particles of the disclosure have an average dimeter or length from about 10 nm to about 100 µm. In a further embodiment, the porous silicon particles of the disclosure have an average dimeter or length from about 10 nm to about 100 nm. In an alternate embodiment, the porous silicon particles have an average dimeter or length from about 100 nm to about 100 µm. In a certain embodiment, the porous silicon material of the disclosure has a porosity from about 50% to about 80%. In another embodiment, the porous silicon material of the disclosure has a porosity of about 75%. In yet another embodiment, the porous silicon material of the disclosure comprises pores that have average diameters from about 2 nm to about 250 nm.

The disclosure provides drug delivery formulations comprising a porous silicon material loaded with a meltable composition; wherein the meltable composition comprises a therapeutic agent and a melting point suppression agent, wherein the therapeutic agent is a contraceptive drug selected from the group consisting of segesterone, etonogestrel, levonorgestrel, levonorgestrel butanoate, and medroxyprogesterone acetate, and wherein the melting point suppression agent is a steroid or a polyketide; wherein the meltable composition has a melting temperature; wherein the therapeutic agent has a melting temperature and a decomposition temperature; and wherein the melting temperature of the meltable composition is lower than the melting temperature of the therapeutic agent and the decomposition temperature of the therapeutic agent.

In other embodiments, the disclosure provides pharmaceutical compositions comprising the drug delivery formulations of the disclosure.

The disclosure provides methods (not claimed) for preparing the drug delivery formulations of the disclosure. In one embodiment, the disclosure provides for a method to make a porous silicon material that have been melt-casted with a thermally unstable therapeutic agent or a mixture thereof, comprising heating a dry mixture comprising: (a) porous silicon material, and (b) an unstable therapeutic agent, or (c) a loading mixture comprising an unstable therapeutic agent and a thermally unstable substance, under an inert atmosphere at a temperature sufficient for melting (b) the unstable therapeutic agent or (c) the mixture comprising the unstable therapeutic agent and a thermally unstable substance, wherein the temperature is not sufficient to cause degradation of the unstable therapeutic agent of (b) or (c); maintaining the porous silicon material with (b) the unstable therapeutic agent or (c) the mixture comprising the unstable therapeutic agent and a thermally unstable substance for a sufficient period time to allow for a mass percentage loading of the pores of the porous silicon material with the melted therapeutic agent, or the melted loading mixture comprising the melted therapeutic agent and the melted substance; cooling the infiltrated porous silicon material to allow for solidification of the melted therapeutic agent, or solidification of the melted loading mixture comprising the melted therapeutic agent and the melted substance; and optionally grinding the cooled infiltrated porous silicon material to generate particles that have average diameters less than 100 µm. In a further embodiment, the porous silicon material are porous materials fabricated by a process comprising: electrochemically or stain etching a crystalline silicon containing substrate to generate a porous silicon material; generating porous layers of the silicon material using electropolishing; collecting, drying and fracturing the layers of the porous silicon material to generate porous silicon microparticles; and oxidizing the porous silicon microparticles in air at a temperature of 800 °C or greater for at least 1 hour or greater to generate porous particles. In an alternate embodiment, the porous silicon material are porous silicon materials fabricated by a process comprising: electrochemically or stain etching a crystalline silicon containing substrate to generate a porous silicon material; generating porous layers of the silicon material using electropolishing; collecting, drying and fracturing the layers of the porous silicon material to generate porous silicon microparticles; and oxidizing the porous silicon microparticles in air at a temperature from 25 °C to 700 °C for 1 hour or less to generate porous particles. In another embodiment, the porous silicon material disclosed herein are loaded with 20% to 90% wt/wt of the melted therapeutic agent or the melted loading mixture comprising the melted therapeutic agent and the melted substance. In yet another embodiment, the porous silicon material disclosed herein are loaded with 50% to 75% wt/wt of the melted therapeutic agent or the melted loading mixture comprising the melted therapeutic agent and the melted substance. In a further embodiment, the porous silicon material disclosed herein have a porosity from about 50% to about 80%. In yet a further embodiment, the porous silicon material disclosed herein have a porosity of about 75%. In a certain embodiment, the porous silicon material disclosed herein comprise pores that have diameters from about 2 nm to about 250 nm. In another embodiment, the porous silicon material disclosed herein comprise pores that have diameters from about 20 nm to about 150 nm. In yet another embodiment, the porous silicon material disclosed herein are particles. In a further embodiment, the porous silicon particles disclosed herein have an average dimeter or length from about 10 nm to about 100 µm. In yet a further embodiment, the porous silicon particles disclosed herein have an average dimeter or length from about 10 nm to about 100 nm.

The invention also provides a pharmaceutical composition comprising the drug delivery formulation of the invention and a pharmaceutically acceptable carrier. The invention also provides a drug delivery formulation according to the invention or the pharmaceutical composition according to the invention, for use in preventing pregnancy in a human female subject in need thereof; preferably, wherein the human female subject is in need of contraception; optionally, wherein the drug delivery formulation or pharmaceutical composition is for parenteral administration; preferably, wherein the drug delivery formulation or pharmaceutical composition is for subcutaneous administration.

In another aspect, the disclosure provides drug delivery formulations comprising a porous silicon material loaded with a progestin drug, wherein the material releases the progestin drug into an aqueous solution over at least 60 days.

### DESCRIPTION OF DRAWINGS

**Figure 1** presents a schematic illustration showing the relationship between composition and morphology for porous silicon. Oxidation of porous silicon prepared by the etching of crystalline silicon induces a decrease in thickness of the silicon skeletal framework (*d*_{*S*i}) and an increase in thickness of the silicon oxide layer (*d*_{SiO2}). The volume expansion associated with conversion of Si to SiO₂ results in a net decrease in average pore diameter (*d*ₚₒᵣₑ).
**Figure 2** provides simultaneous thermal analysis (STA) curves for pure segesterone acetate (SEG) under flowing nitrogen.
**Figure 3** presents steady state release concentrations for pure, non-micronized segesterone acetate at 37°C. Three separate trial runs of the same experiment are shown.
**Figure 4A-B** presents segesterone acetate release profiles of porous silica particles with **(A)** varied oxidation temperature, and **(B)** varied segesterone acetate loading. Each point represents the average of three separate trials and error bars represent one standard deviation.
**Figure 5A-C** presents top-down scanning electron micrographs of **(A)** high porosity, **(B)** medium porosity, and **(C)** low porosity porous silicon. Porous layers were imaged while still adhered to silicon substrates, prior to particle generation. Scale bar represents 500 nm.
**Figure 6** provides steady-state concentrations for segesterone acetate released from 35% porous particles. Three separate trials are indicated.
**Figure 7A-B** provides steady state concentrations for segesterone acetate released from **(A)** 55% porosity, and **(B)** 75% porosity formulations. Three separate trials are indicated.
**Figure 8** demonstrates batch-to-batch variability of segesterone acetate release from 55% porosity particles. Each point represents the average of three trials, with an error bar of one standard deviation.
**Figure 9** presents simulated release profiles for spherical particles and slab-like or tabular particles, assuming both anisotropic and isotropic dissolution models.
**Figure 10A-B** presents **(A)** *in vitro* and **(B)** *in vivo* release curves in rat for 75% porosity, 70 wt% segesterone acetate loaded porous silica particles. Various separate and unique trial (*in vitro*) or animal (*in vivo*) are indicated.
**Figure 11** provides thermal stability evaluation of levonorgestrel, including heat flow (solid) and relative weight (dotted) as temperature increased at a rate of 10 °C in an oxygen atmosphere. Positive spikes in heat flow indicate endothermic processes, such as melting.
**Figure 12** presents the chemical structures of cholesterol, segesterone acetate, and levonorgestrel.
**Figure 13** demonstrates the heating behavior of 100% levonorgestrel (top), 80% levonorgestrel / 20% cholesterol (middle), and 80% levonorgestrel / 20% segesterone acetate (SEG) (bottom) mixtures. Mixtures were heated at a rate of 10 °C under an O₂ atmosphere. Positive heat flow values indicate endothermic processes. Heat flow curves for the three mixtures are offset by arbitrary values to allow for easier interpretation.
**Figure 14** provides HPLC elution curves for pure levonorgestrel (bottom) and levonorgestrel-porous silicon (top), prepared by melt casting with 20% cholesterol. Levonorgestrel was observed to elute from both samples at 6.7 minutes under the same test conditions.
**Figure 15** presents the release kinetics of levonorgestrel from levonorgestrel-porous silicon into 1X PBS (pH 7.4) at 37 °C. Each set of data points (i.e., square, circle, triangle) represents a repeated trial of the same formulation.
**Figure 16** provides the structures of rifampin (left) and rapamycin (right).
**Figure 17** presents the heat flow as a function of temperature for pure RFP (bottom) and an RFP+RAPA mixture (top). Increases in heat flow indicate endothermic activities, such as melting.
**Figure 18A-B** shows differential scanning calorimetry (**A**) and *in vitro* pharmacokinetic release (**B**) of pure segesterone acetate (SEG), SEG loaded into porous silicon via melt casting (MC-SEG) and SEG loaded into porous silicon via evaporation from chloroform (SOLV-SEG).
**Figure 19** shows X-ray diffractograms of SEG (calculated, bottom), SEG (experimental, middle) and SEG melt-casted into 75% porosity porous silicon particles (top).
**Figure 20** shows inverse FWHM for select crystal plane reflections from pure SEG and porous silicon particles loaded with SEG via solvent evaporation and melt casting.

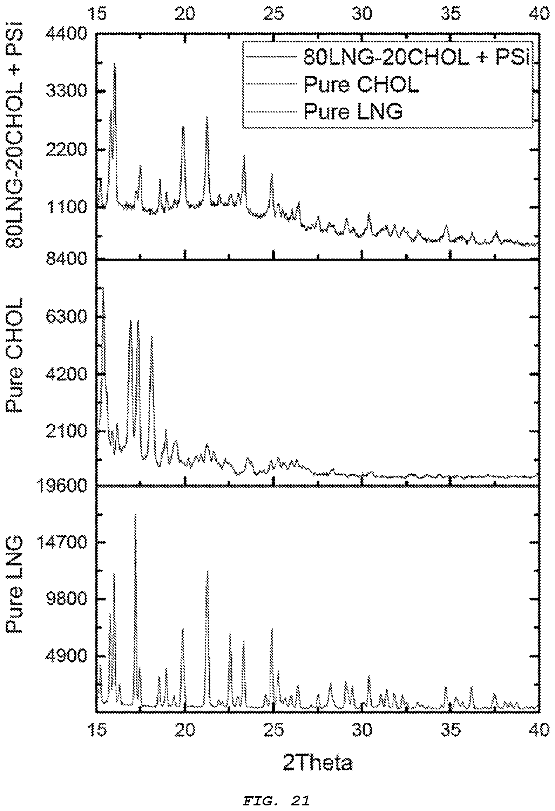

**Figure 21** shows X-ray diffractograms of pure levonorgestrel (LNG, bottom), pure cholesterol (CHOL, middle) and an 80-20 wt% mixture of LNG-CHOL melt casted into porous silicon (PSi) (top).

### DETAILED DESCRIPTION

As used herein and in the appended claims, the singular forms "a," "an," and "the" include plural referents unless the context clearly dictates otherwise. Thus, for example, reference to "a porous silicon particle" includes a plurality of such porous silicon particles and reference to "the melt casting procedure" includes reference to one or more melt casting procedures and equivalents thereof as described herein or understood by one of skill in the art from the disclosure.

Also, the use of "and" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Although many methods and reagents are similar or equivalent to those described herein, the exemplary methods and materials are disclosed herein.

With respect to any term that is presented in one or more publications that is similar to, or identical with, a term that has been expressly defined in this disclosure, the definition of the term as expressly provided in this disclosure will control in all respects.

As used herein, the term "about" or "approximately" means an acceptable error for a particular value, which depends in part on how the value is measured or determined. In certain embodiments, "about" can mean 1 or more standard deviations.

As used herein, the terms "drug" and "therapeutic agent" refer to a compound, or a pharmaceutical composition thereof, which is administered to a subject for treating, preventing, or ameliorating one or more symptoms of a disease, disorder, syndrome, or condition.

As used herein, the term "disorder" is intended to be generally synonymous, and is used interchangeably with, the terms "disease," "syndrome," and "condition" (as in medical condition), in that all reflect an abnormal condition of the body or of one of its parts that impairs normal functioning and is typically manifested by distinguishing signs and symptoms.

As used herein, the term "diluent" defines a solution, typically one that is aqueous or partially aqueous, that stabilizes the biologically active form of the materials, compositions and formulations disclosed herein. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a material, composition or formulation disclosed herein.

As used herein, the term "non-release controlling excipient" refers to an excipient whose primary function do not include modifying the duration or place of release of the therapeutic agent or drug from a dosage form as compared with a conventional immediate release dosage form.

As used herein, the term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenicity, or other problems or complications, commensurate with a reasonable benefit/risk ratio. See, Remington: The Science and Practice of Pharmacy, 21st Edition; Lippincott Williams & Wilkins: Philadelphia, Pa., 2005; Handbook of Pharmaceutical Excipients, 5th Edition; Rowe et al., Eds., The Pharmaceutical Press and the American Pharmaceutical Association: 2005; and Handbook of Pharmaceutical Additives, 3rd Edition; Ash and Ash Eds., Gower Publishing Company: 2007; Pharmaceutical Preformulation and Formulation, Gibson Ed., CRC Press LLC: Boca Raton, Fla., 2004).

As used herein, the terms "prevent," "preventing," and "prevention" refer to a method of delaying or precluding the onset of a disease, disorder, syndrome, or condition; and/or its attendant symptoms, barring a subject from acquiring a disease disorder, syndrome, or condition or reducing a subject's risk of acquiring a disease, disorder, syndrome, or condition.

As used herein, the term "release controlling excipient" refers to an excipient whose primary function is to modify the duration or place of release of the therapeutic agent or drug from a dosage form as compared with a conventional immediate release dosage form.

As used herein, the term "subject" refers to an animal, including, but not limited to, a primate (*e.g.,* human, monkey, chimpanzee, gorilla, and the like), rodents (*e.g.,* rats, mice, gerbils, hamsters, ferrets, and the like), lagomorphs, swine (*e.g.,* pig, miniature pig), equine, canine, feline, and the like. The terms "subject" and "patient" are used interchangeably herein in reference, for example, to a mammalian subject, such as a human subject.

As used herein, the terms "treat," "treating," and "treatment" are meant to include alleviating or abrogating a disease, disorder, syndrome, or condition; or one or more of the symptoms associated with the disorder, disease, syndrome, or condition; or alleviating or eradicating the cause(s) of the disease, disorder, syndrome, or condition itself.

As used herein, the term "therapeutically effective amount" refers to the amount of a drug or therapeutic agent that, when administered, is sufficient to prevent development of, or alleviate to some extent, one or more of the symptoms of the disease, disorder, syndrome, or condition being treated. The term "therapeutically effective amount" also refers to the amount of a drug or therapeutic agent that is sufficient to elicit the biological or medical response of a cell, tissue, system, animal, or human that is being sought by a researcher, veterinarian, medical doctor, or clinician.

Sustained drug delivery began to emerge as a clearly defined sub-area of pharmaceutics in the middle of the twentieth century. The development of the field has been significantly influenced by advances in pharmacokinetics and pharmacodynamics, which served to highlight the need for controlled, extended drug delivery and sustained drug plasma/tissue levels in achieving desired therapeutic responses. In the 1960s and 1970s, companies dedicated to controlled delivery were established (*e.g.,* Alza, Elan). Delivery systems in this field include those that provide zero-order (constant rate) delivery of drugs and sustained-release systems that provide long acting therapy, though not necessarily at a constant rate. Long acting injections and implants can provide systemic, local, or targeted therapy. Delivery systems can also be viewed as macroscale, microscale, or nanoscale.

Formulations and devices for the continuous, long term delivery of therapeutic agents, particularly those formulations and devices that are administered parenterally, have distinct advantages over oral administration or direct injection of the therapeutic agents, since neither of the earlier developed modes can achieve a desired blood level of a drug in circulation for an extended period of time. Oral administration or direct injection bring about a pulse entry of the drug which may create drug concentrations beyond the capacity of the active centers to accept the drug, and may also exceed the capacity of the metabolic and excretory mechanism of the living organism. Thus, if the level of the drug remains elevated, tissues and/or organs may sustain detrimental effects. One technique for reducing excessive concentrations has been to modify the drug structure to provide a longer metabolic half-life; but this in turn has frequently demonstrated lowered therapeutic effectiveness.

To avoid the disadvantages of oral or direct injection administration of drugs, a number of modes of administration of continuous dose, long-term delivery formulations have been used or proposed. These include formulations based upon ingestion, injection, vaginal and uterine insertion, percutaneous application, and subcutaneous implantation. The use of subcutaneous implants offers a particularly desirable combination of properties to permit the administration of substances on a localized or systemic basis. To this end, subcutaneous implants serving as depots capable of slow release of a drug have been proposed. These implants suggest the possibility of attaining continuous administration over a prolonged period of time to achieve a relatively uniform delivery rate and, if desired, astatic blood level. Since an excessive concentration of drug never enters the body fluids, problems of pulse entry are overcome and metabolic half-life is not a factor of controlling importance.

Despite the advantages of administering drugs from implants, prior art formulations and devices designed for this purpose have possessed one or more disadvantages which limit their acceptability and efficacy. Among such disadvantages are non-biodegradability which may require a surgical procedure to remove any residual components; non-biocompatibility which may result in the introduction of undesirable and even harmful substances into the body; antigenicity which gives rise to the production of unwanted antigen bodies in the system; and difficulty in controlling the release rates of the drugs.

The use of porous silicon and silicon oxide materials, including so-called "smart dust" photonic crystal nanoparticles, for drug delivery have been described WO2014/130998; WO2017/008059; and WO2017/181115. See also Salonen et al. (2008) J. Pharm. Sci. 97:632 and Anglin et al. (2008) Adv. Drug Delivery Rev. 60:1266. Porous silicon materials are especially advantageous for these purposes, due to their large free volume (typically 50-80%) and consequent high capacity for drug loading. Their relatively slow rates of dissolution under physiological conditions, and their conversion to relatively non-toxic silicic acid, further highlight the advantages of porous silicon materials in the delivery of therapeutic agents over long time periods with low toxicity, and further illustrate the benefits of these materials as drug delivery vehicles.

Typically porous silicon materials are loaded with therapeutic agents by dissolving the agent in an appropriate solvent, incubating the porous silicon material with the dissolved agent, and thereby allowing the agent to be taken up by the porous silicon material. However, not all therapeutic agents are soluble in an appropriate solvent at sufficient concentrations. The extent of loading using these techniques can therefore be lower than desired. An alternative approach for loading these materials at higher levels involves melting the solid therapeutic or other agent, exposing the porous silicon material to the molten agent, and thereby allowing the agent to be taken up by the porous silicon material. Such "melt casting", "melt loading", or "melt adsorption" techniques have been used, for example, to load triclosan and TAS-301 into porous silicon materials. See, Wang et al. (2010) Mol. Pharmaceutics 7:2232 and Kinoshita et al. (2002) J. Pharma. Sci. 91:362. The techniques are not, however, considered suitable where the melting temperature of the therapeutic agent is close to the decomposition temperature of the agent, since the agent is not sufficiently stable to be loaded into the porous material in the liquid phase. The release profiles of therapeutic agents that have been melt cast into porous silicon materials may also not be suitable for the desired therapeutic use.

Microparticles, microspheres, and microcapsules, referred to herein collectively as "microparticles", are solid or semi-solid particles having a diameter of less than one millimeter, typically less than 100 microns, which can be formed of a variety of materials, including synthetic polymers, proteins, and polysaccharides. Microparticles have been used in many different applications, primarily separations, diagnostics, and drug delivery. In the controlled drug delivery area, therapeutic molecules are encapsulated within microparticles or incorporated into a monolithic matrix, for subsequent release. A number of different techniques are routinely used to make these microparticles from synthetic polymers, natural polymers, proteins and polysaccharides, including phase separation, solvent evaporation, emulsification, and spray drying. Generally, the polymers form the supporting structure of these microspheres, and the therapeutic agent of interest is incorporated into the polymer structure. Exemplary polymers used for the formation of microspheres include homopolymers and copolymers of lactic acid and glycolic acid (PLGA). Microspheres produced using polymers such as this exhibit a poor loading efficiency, however, and are often only able to incorporate a small percentage of the drug of interest into the polymer structure. Therefore, substantial quantities of microspheres often must be administered to achieve a desired therapeutic effect.

One further disadvantage of the microparticles or beads currently available is that they are difficult and expensive to produce. Microparticles produced by these known methods have a wide particle size distribution, often lack uniformity, and fail to exhibit long term release kinetics when the concentration of active ingredients is high. Residual organic solvents could be toxic when administered to humans or animals.

Microparticles prepared using lipids to encapsulate target drugs are also currently available. For example, liposomes are spherical particles composed of a single or multiple phospholipids and cholesterol bilayers. Liposome technology has been hindered by problems including purity of lipid components, possible toxicity, vesicle heterogeneity and stability, excessive uptake, and manufacturing or shelf-life difficulties. Therefore, there is an ongoing need for the development of new formulations for long-term drug delivery. Preferably, such improved formulations will achieve the sustained release of therapeutic levels of active therapeutic agents in a predictable and consistent manner.

The instant disclosure provides novel drug delivery formulations having these desirable properties and lacking the undesirable biphasic release properties (*e.g.,* burst release followed by long tail release) common in other drug delivery formulations and devices. In particular, the disclosed compositions and formulations comprise a porous silicon material loaded with a meltable composition. As used herein "a porous silicon material" refers to a material that is comprised of silicon, typically crystalline silicon, which has been treated by a process that has introduced a plurality of void spaces or pores into the material. A "porous silicon material" can further comprise some limited portion of which that is not silicon, *e.g.,* carbon. A "porous silicon material" can comprise any shape or shapes. For example, a "porous silicon material" can include porous silicon films, porous silicon layers, porous silicon particles, etc., as will be described in more detail below.

In some embodiments, the porous silicon material of the disclosure can comprise silicon oxide or silicon dioxide. For purposes of this disclosure, a "porous silicon dioxide material" or a "porous silica material" refers to a porous silicon material that comprises at least some silicon oxide or silicon dioxide. In specific embodiments, the porous silicon material has been oxidized so that one or more surfaces of the porous silicon material comprises silicon dioxide or silica. As such, "porous silicon dioxide" or "porous silica" refers to porous silicon materials or particles that have been oxidized using methods disclosed herein or in the art, so that materials or particles comprise up to a significant portion of which is silicon oxide. A "porous silica material" may further comprise some limited portion of which that is not silica, *e.g.,* silicon.

In some embodiments, the porous silicon material can be prepared using a sol-gel process from appropriate soluble precursors. However, the porosity of such porous silicon materials is not as well controlled as materials formed by the etching of crystalline silicon. Where the porous silicon oxide or porous silicon dioxide is prepared using a sol-gel process, the resultant porous silicon material may be referred to as a silica gel.

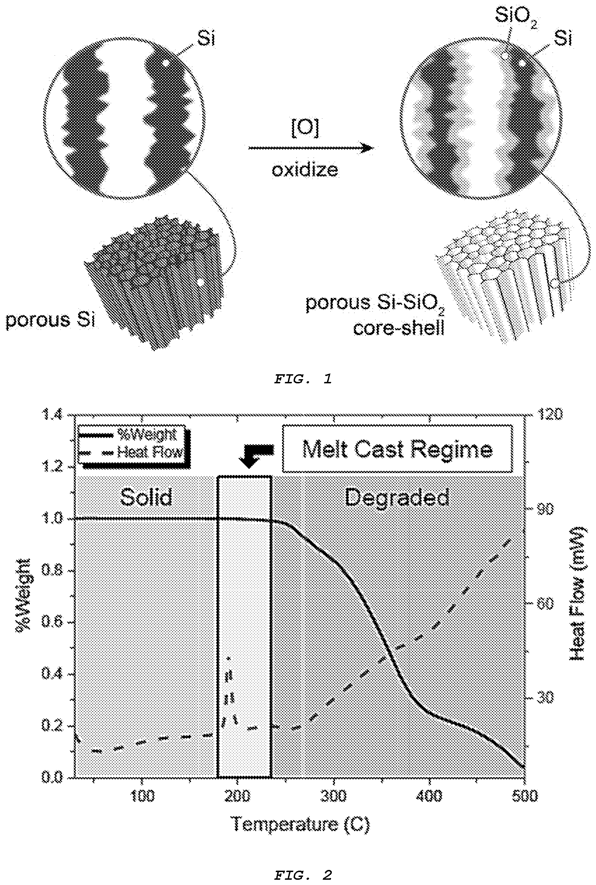

As used herein a "porous silicon material" refers to a material that is comprised of silicon and optionally other elements, and which comprises a plurality of void spaces or pores in the material. For example, a "porous silica material" as used herein, can comprise structures that have a core of silicon with an outer sheath of silica (*e.g.,* see **FIG. 1**). In this context, a "porous silicon material" refers to a porous silicon-based material that contains a portion of silicon that is in its elemental form, that is, not oxidized, and a portion of silicon that has been oxidized so that one or more surfaces of the porous silicon material comprises silicon dioxide. Additionally, and as mentioned above, a "porous silicon material" may further comprise some limited portion of which is not silicon, *e.g.,* carbon.

Typically, for the porous silicon materials described herein, the void spaces or pores are between 1 nm to 100 µm in diameter or size. The dimensions of the void spaces or pores are generally tunable, *i.e.,* the dimensions of the pores of the porous silicon material can be controlled to have a pore size dimension (*e.g.,* diameter) of 1 nm, 5 nm, 10 nm, 15 nm, 20 nm, 25 nm, 30 nm, 35 nm, 40 nm, 45 nm, 50 nm, 55 nm, 60 nm, 70 nm, 80 nm, 100 nm, 110 nm, 120 nm, 130 nm, 140 nm, 150 nm, 200 nm, 300 nm, 400 nm, 500 nm, 1 µm, 10 µm, 50 µm, 100 µm, or any range of pore sizes that includes or is between any two of the foregoing percentages, including fractions thereof. The pore sizes can also be varied in diameter (*e.g.,* one side of a porous silicon material may have substantially the same size pores while the opposite side of the porous silicon material may have pores that are substantially larger, by about 25% or greater). Additionally, the overall porosity density of the porous silicon material can be controlled using methods disclosed herein, such that extremely porous silicon materials to not so porous silicon materials can be made. In certain embodiments, the porous silicon material can have a porosity of 5%, 10%, 15%, 20%, 25%, 30%, 35%, 40%, 45%, 50%, 55%, 60%, 65%, 70%, 75%, 80%, 85%, 90%, 95%, or any range of porosity that includes or is between any two of the foregoing percentages, including fractions thereof. Porous silicon structures can easily withstand temperatures well in excess of what is required for wet or dry heat sterilization. For example, the porous silicon materials of the disclosure readily tolerate heat sterilization without impacting their physical properties. Porous silicon materials have low toxicity profiles and excellent drug delivery properties. See, *e.g.,* PCT International Publication Nos. WO2006/050221 and WO2009/009563. Under biological conditions, the primary degradation product of these materials is orthosilicic acid, a non-toxic water-soluble compound naturally found in human tissues that is readily cleared through the renal system.

The dramatic improvement in pharmacokinetic profile using porous silicon materials that have been prepared from crystalline silicon is directly linked to the highly aligned nature of the pores in this material, which results in anisotropic dissolution of the drug-carrier matrix. In these materials, the carrier dissolves preferentially along the pore direction, guiding drug release into a zero-order kinetic regime that result in a linear release profile not seen with crystalline DMPA or with polymeric drug delivery systems. Free drug concentration is more constant throughout the release period, and the drug concentration rapidly tapers at the end. Furthermore, by minimizing the initial burst release of the drug, a larger dose could potentially be administered without significant side effects, enabling 6-months or even 1-year formulations.

In the instant drug delivery formulations, the porous silicon material is loaded with a meltable composition, wherein the meltable composition comprises a therapeutic agent and a melting point suppression agent. As would be understood by those of ordinary skill in the art, the meltable composition has a melting temperature, and the separate therapeutic agent, not part of the meltable composition, has a melting temperature and a decomposition temperature. All of these temperatures can be determined using standard analytical chemical techniques. For example, and as described in more detail in the examples section, both heat flow and relative weight can be measured for a given sample as a function of temperature. The technique is referred to herein as simultaneous thermal analysis (STA). Increases in heat flow as the sample is heated indicate endothermic processes, such as melting. Decreases in relative weight as the sample is heated indicate decomposition. Samples that decompose at temperatures in the vicinity of their melting temperature are not good candidates for melt cast loading into a porous silicon material, since the window of thermal stability in the liquid phase is too narrow, and the sample will not flow into the porous silicon material before it decomposes.

Previously, therapeutic agents that decomposed at temperatures close to their melting temperatures were understood to be poor candidates for melt casting into porous silicon materials. See, e.g., U.S. Patent Nos. 8,088,401 and 9,243,144. This disclosure shows, however, that certain agents, to be referred to herein as "melting point suppression agents", can decrease the temperature necessary to melt a composition comprising the therapeutic agent. The melting point suppression agent thus enlarges the window of temperatures available for melt casting of therapeutic agents into a porous silicon material. In some embodiments, the meltable composition can be described as a eutectic mixture, where the eutectic mixture exhibits a depressed melting point. A eutectic mixture is typically understood to be an intimately blended physical mixture of two or more crystalline components that melts as a single phase, having a melting point lower than that of either or any of the separate components. Without intending to be bound by theory, a eutectic mixture is thought to form when the two (or more) different crystalline components are mismatched in terms of molecular size or molecular shape, such that cohesive interactions are relatively stronger than adhesive interactions, thus leading to a conglomerate of the two or more lattice structures rather than a new lattice structure. At the same time, however, it is generally understood that there are no ground rules or structural guidelines as to the point at which the cohesive interactions dominate over the adhesive interactions (to give a eutectic) and vice versa (to give a co-crystal). Accordingly, it should be noted that the exact structural nature of the meltable composition of the disclosure (*i.e.,* eutectic, co-crystal, or mixture thereof) need not be determined for the purposes of working the invention, as the key feature that all of the embodiments above share is that of having a depressed melting point. Furthermore, the difference in temperature between the depressed melting point of the meltable composition and the decomposition temperature of the therapeutic agent can readily be determined experimentally using, for example, simultaneous thermal analysis and other analogous techniques.

Examples of therapeutic agents that have previously been demonstrated to usefully form eutectic mixtures include without limitation paclitaxel (U.S. Patent Application Publication No. 2017/0360991A1), lorazepam (U.S. Patent Application Publication No. 2016/0000702A1), and various local anesthetics (U.S. Patent No. 4,562,060). In addition to their improved ability to be melt cast into porous silicon materials, eutectic mixtures can have other advantages, such as higher or lower rates of dissolution and improved bioavailability of the therapeutic agent.

As would be understood from the above description, the smaller the difference between the melting temperature of the therapeutic agent and the decomposition temperature of the therapeutic agent, the more important it can be to include a melting point suppression agent in the meltable composition. Accordingly, in some embodiments, the melting temperature of the therapeutic agent is no more than about 50 °C, 20 °C, 10 °C, 5 °C, 2 °C, or even 1 °C lower than the decomposition temperature of the therapeutic agent. In specific embodiments, the melting temperature of the therapeutic agent is no more than about 5 °C, 2 °C, or even 1 °C lower than the decomposition temperature of the therapeutic agent.

Likewise, the larger the difference between the melting temperature of the meltable composition and the decomposition temperature of the therapeutic agent, the better the chances of achieving the desired melt cast formulation without decomposition of the therapeutic agent. Accordingly, in some embodiments, the melting temperature of the meltable composition is at least about 1 °C, 2 °C, 5 °C, 10 °C, 20 °C, or even 50 °C lower than the decomposition temperature of the therapeutic agent. It should also be understood, however, that it is not necessary to determine the melting temperature of the meltable composition, so long as the melt cast formulation can be prepared without significant decomposition of the therapeutic agent during the process.

The steroidal ring system (shown below) is a well-known and well-understood structural framework for a large variety of naturally-occurring and man-made molecules, including many hormones and other agents with various important structural and functional properties in biological systems. 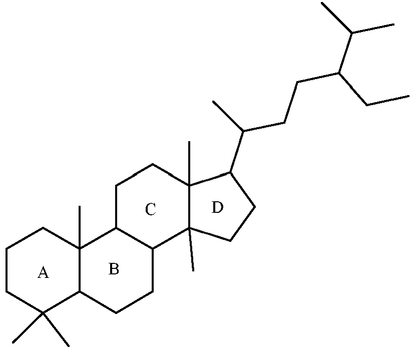 In particular, many contraceptive agents are steroidal drugs. Accordingly, in the instant drug delivery formulations, the therapeutic agent of the meltable composition is a contraceptive drug. In some embodiments, the contraceptive drug includes two or more separate active agents, including two or more steroidal drugs. For example, some birth control pills contain both an estrogen and a progestogen, each of which corresponds to a different class of steroidal drug. In some cases, a contraceptive drug may comprise only one active agent, for example a progestogen. Exemplary contraceptive drugs include, without limitation, estradiol, ethinylestradiol, progesterone, noretynodrel, etynodiol diacetate, norethisterone (also known as norethindrone), lynestrenol, racemic norgestrel, levonorgestrel, desogestrel, nomegestrol acetate, norgestimate, dienogest, etonogestrel, drospirenone, norelgestromin, and segesterone acetate (also known as nestorone, sometimes abbreviated SEG).

In the meltable compositions, the therapeutic agent is selected from the group consisting of segesterone, etonogestrel, levonorgestrel, levonorgestrel butanoate, and medroxyprogesterone acetate. In some embodiments, the therapeutic agent is etonogestrel or levonorgestrel.

Polyketides represent a structurally diverse family of natural products having various biological and pharmacological behaviors. They typically occur biosynthetically through the decarboxylative condensation of malonyl-CoA-derived extender units, as is well understood in the art, so they share certain structural and functional features. It should also be understood that included in the definition of polyketides are natural products that have been further derivatized and/or modified into bioactive molecules.

From a structural perspective, polyketides include without limitation ansamycins, macrolides, polyethers, polyenes, tetracyclines, and acetogenins. From a functional perspective, polyketide therapeutic agents include without limitation antibiotics, anticholesteremics, antifungals, antiparasitics, antiprotozoals, cytostatics, and animal growth promoters. Exemplary polyketide therapeutic agents useful in the drug delivery formulations of the disclosure include without limitation the rifamycins, for example, the naturally-occurring rifamycins, rifaximin, rifalazil, rifapentine, ribabutin, and rifampin; geldanamycin; macbecin; amphotericin; nystatin; pimaricin; monensin; doxycycline; various immunosuppressants, including tacrolimus and sirolimus (rapamycin); lovastatin; erythromycin A; clarithromycin; azithromycin; avermectin; ivermectin; and spinosad (spinosyn).

In addition to a therapeutic agent, the meltable compositions according to the disclosure comprise a melting point suppression agent. Any suitable compound as defined herein may function as a melting point suppression agent in the meltable compositions, so long as the melting temperature of the meltable composition is lower than melting temperature of the pure therapeutic agent and is also lower than the decomposition temperature of the therapeutic agent separate from the melting point suppression agent. In addition, the presence of the melting point suppression agent in the meltable composition should preferably not significantly interfere with the therapeutic effectiveness of the therapeutic agent. In some embodiments, the melting point suppression agent may itself have desirable therapeutic properties either in addition to, or separate from, the therapeutic agent of the meltable composition. In other words, the melting point suppression agent may itself also serve as a therapeutic agent.

The presence of the melting point suppression agent in the meltable composition may additionally, in some cases, improve the release profile of the resulting drug delivery formulation by, for example, altering the packing structure of the meltable composition as it solidifies in the porous silicon material, compared to the packing structure of the pure, solid therapeutic agent, so that the therapeutic agent in the drug delivery formulation is released either faster or slower than it would otherwise be released.

In some embodiments, the melting point suppression agent has a generally similar structure to the therapeutic agent. The melting point suppression agent may be a steroid. In certain embodiments, the melting point suppression may, for example, be cholesterol. The melting point suppression agent may be a polyketide. In certain embodiments, the melting point suppression agent may, for example, be rapamycin or another polyketide compound.

In an embodiment not according to the invention, the drug delivery formulation of the disclosure comprises a porous silicon material loaded with a progestin drug and no melting point suppression agent. In certain embodiments of the disclosure, the material releases the progestin drug into an aqueous solution, for example the bloodstream of a female human subject treated with the formulation, over at least 60 days. As demonstrated herein, the release profile of a progestin drug, for example segesterone acetate, that has been melt cast into the porous silicon materials of the disclosure, is surprisingly better than that of existing progestin drug formulations, particularly in terms of lower burst release, longer sustained release, and less long tail behavior.

In these embodiments, the porous silicon material is an oxidized porous silicon material, and is typically an oxidized porous silicon material that has been oxidized at a temperature of 800 °C or greater for 1 hour or longer.

In some embodiments, the progestin drug is selected from the group consisting of medroxyprogesterone acetate (MPA), levonorgestrel butanoate (LNG-B), and segesterone acetate (NES). Even more specifically, the progestin drug is segesterone acetate.

In some embodiments, the porous silicon material releases the progestin drug into an aqueous solution over at least 80 days, 100 days, 120 days, or even longer.

The porous silicon materials can be nano- to micro-meter sized porous silicon particles. Such particles can conveniently be made from a porous silicon source material (*e.g.,* a porous silicon wafer), for example by fracturing. In some embodiments, the porous silicon materials are nano- to milli-meter sized particles. For example, the porous silicon particles can have an average diameter (or length dimension) of 3 nm, 4 nm, 5 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 100 nm, 150 nm, 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, 1 µm, 2 µm, 3 µm, 4 µm, 5 µm, 6 µm, 7 µm, 8 µm, 9 µm, 10 µm, 15 µm, 20 µm, 25 µm, 30 µm, 35 µm, 40 µm, 45 µm, 50 µm, 55 µm, 60 µm, 65 µm, 70 µm, 80 µm, 85 µm, 90 µm, 95 µm, 100 µm, 150 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, or a range that includes or is between any two of the foregoing values, including fractions thereof. Moreover, the porous silicon particles can have regular shapes, e.g., generally spherical, cuboidal, polyhedral, cylindrical, conical, cubic, ellipsoidal, pyramidal, etc., or have irregular shapes.

In some embodiments, electrochemical or chemical etching of crystalline silicon wafers using methods known in the art can provide for porous silicon materials that comprise an array of pores on the order of a few nanometers in diameter. In regards to the foregoing embodiments, the pores are typically highly ordered, and generally oriented along the <100> crystallographic direction of the wafer. The porous silicon material disclosed herein can be converted or modified to comprise porous silicon dioxide (silica), for example by thermal oxidation. As indicated further herein, the temperature at which the thermal oxidation is carried out can greatly affect the composition of the resulting silica material. For example, the use of oxidation temperatures less than about 800 °C for less than about an hour can give rise to porous silicon materials that are not fully oxidized. Such material may, for example, comprise a core of silicon with a coating of a defined or undefined thickness of silica (see, *e.g.,* **FIG. 1**). Ideally, however, the oxidized porous silicon material is prepared by the complete oxidation of porous silicon in an oxidizing environment.

In certain embodiments, a multilayered porous nanostructure can be produced from a silicon wafer or chip by using pulsed electrochemical etching. See, *e.g.,* PCT International Publication Nos. WO2006/050221 and WO2009/009563.

The thickness, pore size, porosity, and surface area of a given porous silicon material can be generally controlled based upon the specific process used. For example, tunability for an etching process results from the current density used, duration of the etch cycle, and etchant solution composition. In addition, a porous silicon material can be used as a template to generate an imprint of biologically compatible or bioresorbable materials. The porous silicon material or its imprint possess a sinusoidally varying porosity gradient, providing sharp features in the optical reflectivity spectrum that can be used to monitor the presence or absence of chemicals trapped in the pores. It has been shown that the particles made from a porous silicon material, such as by mechanical grinding or by ultrasonic fracture, still comprise the optical reflectivity spectrum.

In some embodiments, a porous silicon material results from electrochemical anodization of single crystalline silicon wafer in a hydrofluoric acid electrolyte solution. Pore morphology and pore size can be varied by controlling the current density, the type and concentration of dopant, the crystalline orientation of the wafer, and the electrolyte concentration in order to form micro- and/or nano-sized pores. In general, the relationships of dopant to morphology can be segregated into four groups based on the type and concentration of the dopant: n-type, p-type, highly doped n-type, and highly doped p-type. By "highly doped," is meant dopant levels at which the conductivity behavior of the material is more metallic than semiconducting. For n-type silicon wafers with a relatively moderate doping level, exclusion of valence band holes from the space charge region determines the pore diameter. Quantum confinement effects are thought to limit pore size in moderately p-doped material. For both dopant types the reaction can be crystal face selective, with the pores propagating primarily in the <100> direction of the single crystal. For example, electrochemically driven reactions use an electrolyte containing hydrofluoric acid. Application of anodic current oxidizes a surface silicon atom, which is then attacked by fluoride. The net process is a 4-electron oxidation, but only two equivalents are supplied by the current source. The other two equivalents come from reduction of protons in the solution by surface SiF₂ species. Pore formation occurs as silicon atoms are removed in the form of SiF₄, which reacts with two equivalents of F- in solution to form SiF₆²⁻.

The porosity of a growing porous silicon layer is understood to be proportional to the current density being applied, and it typically ranges between 40% and 80%. Pores form at the silicon/porous silicon interface, and once formed, the morphology of the pores does not change significantly for the remainder of the etching process. However, the porosity of a growing layer can be altered by changing the applied current. The film will continue to grow with this new porosity until the current changes.

Stain etching is an alternative to the electrochemical method for fabrication of porous silicon-based materials. The term "stain etching" refers to the brownish or reddish color of the porous silicon material generated from a crystalline silicon material subjected to the process. In the stain etching procedure, a chemical oxidant (typically nitric acid) replaces the power supply used in the electrochemically driven reaction. HF is typically used as an ingredient, and various other additives are used to control the reaction.

For *in vivo* applications, it is often desirable to prepare porous silicon materials in the form of particles. The porous layer can be removed from the silicon substrate with a procedure commonly referred to as "electropolishing" or "lift-off." The etching electrolyte is replaced with one containing a lower concentration of HF and a current pulse is applied for several seconds. The lower concentration of HF results in a diffusion limited situation that removes silicon from the crystalline silicon/porous silicon interface faster than pores can propagate. The result is an undercutting of the porous layer, releasing it from the silicon substrate. The freestanding porous silicon material can then be removed with tweezers or a vigorous rinse. The material can then be converted into particles by ultrasonic fracture. Typically, micron sized particles result. Conventional lithography or microdroplet patterning methods can also be used if particles with more uniform shapes are desired.

The ability to easily tune the pore sizes and volumes during the electrochemical etch is a unique property of porous silicon that is very useful for drug delivery applications. Other porous non-silicon materials generally require a more complicated design protocol to control pore size, and even then, the available pore sizes tend to span a limited range. With electrochemically prepared porous silicon materials, control over porosity and pore size is obtained by adjusting the current settings during the etch. Typically, larger current density produces larger pores. Large pores are desirable when incorporating sizable molecules or drugs within the pores. Pore size and porosity is important not only for drug loading; but it also determines degradation rates of the porous silicon particles. Smaller pores provide more surface area and expose more sites for attack by aqueous media.

Surface chemistry plays a large role in controlling the degradation properties of porous silicon *in vivo.* Immediately after silicon is electrochemically etched, the surface is covered with reactive hydride species. These chemical functionalities provide a versatile starting point for various reactions that determine the dissolution rates in aqueous media, allow the attachment of homing species, and control the release rates of drugs. The two most important modification reactions are chemical oxidation and grafting of silicon-carbon species.

With its high surface area, porous silicon is particularly susceptible to air or water oxidation. Once oxidized, nanophase silicon dioxide readily dissolves in aqueous media, and surfactants or nucleophiles accelerate the process. Silicon-oxygen bonds are easy to prepare on porous silicon by oxidation, and a variety of chemical or electrochemical oxidants can be used. Thermal oxidation in air tends to produce a relatively stable oxide, in particular if the reaction is performed at >800 °C for 1 hour or greater. Ozone oxidation, usually performed at room temperature, forms a more hydrated oxide that dissolves quickly in aqueous media. Milder chemical oxidants, such as dimethyl sulfoxide, benzoquinone, or pyridine, can also be used for this reaction. Mild oxidants are sometimes used because they can improve the mechanical stability of highly porous silicon materials, which can be fragile. In a particular embodiment, the disclosure provides for porous silicon materials or particles that have been thermally oxidized in the presence of an oxidant (e.g., air) at a temperature of 400 °C, 450 °C, 500 °C, 550 °C, 600 °C, 650 °C, 700 °C, 750 °C, 800 °C, 850 °C, 900 °C, 950 °C, 1000 °C, 1050 °C, 1100 °C, 1150 °C, 1200 °C, or any range of temperatures that includes or is between any two of the foregoing temperatures, including fractions thereof; and maintained at the foregoing temperature(s) for 1 min, 5 min, 10 min, 15 min, 20 min, 25 min, 30 min, 35 min, 40 min, 45 min, 50 min, 55 min, 1 h, 1.5 h, 2 h, 3 h, 4 h, 5 h, 10 h, 16 h, 24 h, or any range of time periods that includes or is between any two of the foregoing stated times, including fractions thereof. Although porous silicon is converted to porous silica by thermolysis in air, if the process is not carried to completion there is substantial elemental silicon remaining in the porous silica material. Incomplete thermal oxidation results in formation of an oxide layer on the outer surface of the porous silicon skeleton, resulting in Si-SiO₂ skeleton-sheath types of structures (see **FIG. 1**). Heat treatment in air accomplishes two separate processes: (1) at lower temperatures it induces oxidation, forming a silica sheath around the elemental silicon skeletal framework; and (2) at temperatures >950 °C the silica layer softens and flows. The thickness of the oxide sheath increases from a relatively thin value for samples heated at lower temperatures (or merely stored at ambient temperatures for a period of time) to thicker values at higher temperatures and ultimately (at temperatures >800 °C) to a state where the nanostructure is completely converted to oxidized silicon (i.e., silica). The oxide sheath has been observed to increase the chemical stability and biocompatibility, decrease the refractive index and the absorption coefficient, and improve the photo-luminescent properties of the porous material. The extent of oxidation depends on not only the temperature but also the time the sample is held at temperature. It is widely accepted that porous silicon samples that contain relatively thin (10 nm) pore walls can be fully oxidized to porous silica by treatment in ambient air at 800 °C for 1 h. The possible collapse of the porous structures due to softening or melting of SiO₂ is a significant concern, because much interest in porous silicon as a drug carrier focuses on its ability to retain large amounts of drug within the porous matrix and to controllably deliver the drug *in vivo.* Viscous flow of the oxide has been reported to begin at temperatures >960 °C. Even at temperatures where the pores do not collapse, the volume increase upon conversion of Si to SiO₂ decreases both porosity and average pore size.

The mechanical instability of porous silicon is directly related to the strain that is induced in the material as it is produced in the electrochemical etching process, and the volume expansion that accompanies thermal oxidation can also introduce strain. Mild chemical oxidants presumably attack porous Si typically at Si-Si bonds that are the most strained, and hence most reactive. As an alternative, nitrate is a stronger oxidant, and nitric acid solutions are used extensively in the preparation of porous Si particles from silicon powders by chemical stain etching.

Slow oxidation of the porous silicon surface by dimethyl sulfoxide (DMSO), when coupled with dissolution of the newly formed oxide by HF, is a mild means to enlarge the pores in porous silicon materials. Aqueous solutions of bases such as potassium hydroxide can also be used to enlarge the pores after etching. Electrochemical oxidation, in which a porous silicon material is anodized in the presence of a mineral acid such as H₂SO₄, yields a fairly stable oxide. Oxidation imparts hydrophilicity to the porous structure, enabling the incorporation and adsorption of hydrophilic drugs or biomolecules within the pores. Aqueous oxidation in the presence of various ions including Ca²⁺ generates a calcified form of porous silicon that has been shown to be bioactive and is of particular interest for *in vivo* applications. Calcification can be enhanced by application of a DC electric current.

Carbon grafting stabilizes porous silicon against dissolution in aqueous media, but the surface must still avoid the non-specific binding of proteins and other species that can lead to opsonization or encapsulation. Reactions that place a polyethylene glycol (PEG) linker on a porous silicon surface have been employed to this end. A short-chain PEG linker yields a hydrophilic surface that is capable of passing biomolecules into or out of the pores without binding them strongly. The distal end of the PEG linker can be modified to allow coupling of other species, such as drugs, cleavable linkers, or targeting moieties, to the material.

Oxides of porous silicon are easy to functionalize using conventional silanol chemistries. When small pores are present (as with p-type samples), monoalkoxydimethylsilanes (RO-Si(Me)₂-R') can be more effective than trialkoxysilanes ((RO)₃Si-R') as surface linkers. This is because trialkoxysilanes oligomerize and clog smaller pore openings, especially when the reagent is used at higher concentrations.

Whereas Si-C chemistries are robust and versatile, chemistries involving Si-O bonds represent an attractive alternative for two reasons. First, the timescale in which highly porous silica is stable in aqueous media is consistent with many short-term drug delivery applications-typically 20 min to a few hours. Second, a porous silica sample that contains no additional stabilizing chemistries is less likely to produce toxic or antigenic side effects. If it is desired that the porous silicon material be stable *in vivo* for long periods (for example, an extended release formulation or an *in vivo* biosensor), silicon-carbon chemistries such as hydrosilylation with endcapping or thermal carbonization with acetylene is useful. If a longer-lived oxide matrix is desired, silicon oxides formed at higher temperatures (>700 °C) are significantly more stable in aqueous media than those formed at lower temperatures or by ozone oxidation.

Further, porous silicon dioxide can comprise a higher concentration of a therapeutic agent than a non-oxidized silicon hydride material. For example, the oxide treatment causes the oxidized porous silicon material to absorb larger quantities of the therapeutic agent than are absorbed by the freshly prepared (hydride-terminated) porous silicon material.

The void volume in a porous silicon material is typically between 20% and 80%, but can be greater percentage or a lesser percentage is so desired. Oxidation should reduce this value somewhat, but the free volume remains high. Most of the current drug delivery materials are dense solids and can deliver a small percentage of drug by weight. The amount of drug that can be loaded into the porous silicon materials is expected to be much larger than, for example, surface-modified nanoparticles or polylactide (PLA) polymers. Experiments can quantify the amount of each of the drugs that can be loaded into the porous silicon materials of the disclosure.

During chemical modification, a molecule is attached to the inner pore walls via covalent bonds. For the porous silicon materials disclosed herein, proteins, DNA, and various small molecules can be attached following several different procedures. One embodiment uses electrochemical modification. For example, reduction of 1-iodo-6-(trifluoroacetylamino) hexane at a p-type porous silicon cathode leads to attachment of the trifluoroacetamidohexyl group. Subsequent acid-catalyzed hydrolysis should lead directly to the surface-bound amine species. The surface amine can then be functionalized with a drug, polypeptide or peptide. For example, the surface amine can be functionalized with a peptide fragment using standard peptide coupling methods.

While various methods can be used to load the porous silicon materials with one or more drug products (*e.g.,* covalent attachment, physical trapping, and adsorption), the use of a melt casting approach has been found to be especially beneficial. In particular, it was found that the nanostructure of the porous silicon materials of the disclosure was retained by using a melt casting approach. A comparison of the use of melt casting and solution casting methods (also referred to as melt processing and solution processing) in the generation of organic and bio-polymer nanostructures on porous silicon templates is provided in U.S. Patent No. 7,713,778. In those uses, the porous silicon template is removed following the casting process to leave a residual organic or bio-polymer nanostructure, whereas the formulations provided herein for long-term drug delivery retain the porous silicon material as part of the final drug-delivery formulation.

Further studies clearly establish the viability of using melt casting as a method to load drugs and therapeutic agents into porous silicon materials. In particular, it was found that therapeutic agents and drugs, like progestin drugs (*e.g.,* medroxyprogesterone acetate (MPA), levonorgestrel butanoate (LNG-B), Segesterone acetate (SEG)), could be loaded into the porous silicon particles at high mass loadings. Thermogravimetric and differential thermal analyses showed that the tested drugs were not degraded when melt casting was performed in an inert (N₂ or Ar) atmosphere. HPLC and mass spectral analyses of the drugs released from the porous silicon particles confirmed that melt casting did not chemically degrade the drugs. It was found, however, that use of a direct melt casting procedure may not be appropriate for use with pure drug products or therapeutic agents that have degradation temperatures that closely align with their melting points. For example, levonorgestrel (LNG) and etonogestrel (ENG) were both found to thermally degraded at temperatures only 2 or 3°C, respectively, above their melting points. This narrow window of liquid stability was insufficient to allow melt casting of the pure drug substances without degradation. The disclosure thus provides a technical solution for the foregoing narrow window technical problem by employing a meltable composition that comprises the therapeutic agent, in particular a thermally unstable therapeutic agent, with a melting point suppression agent.

In particular, it was found herein that thermally unstable drugs, which have a melting temperature that is closely aligned with their degradation temperature (also referred to herein as a decomposition temperature), can be melt cast into the porous silicon materials of the disclosure by using mixtures which comprise the therapeutic agent with one or more other compounds that are melting point suppression agents. The use of one or more melting point suppression agents with the thermally unstable therapeutic agent lowers the melting point of the combined meltable composition, thus expanding the window between the melting point of the meltable composition and the degradation temperature of the therapeutic agent. The combination thus allows for the melt casting of therapeutic agents into a porous silicon material that would not otherwise be possible.

In an exemplary embodiment presented herein, a model therapeutic agent, levonorgestrel, was mixed with a melting point suppression agent, cholesterol, thus resulting in a meltable composition that is capable of being loaded into a porous silicon material without the decomposition of the therapeutic agent. Absent the melting point suppression agent, melt casting of levonorgestrel into a porous silicon material would be impossible due to its thermal instability.

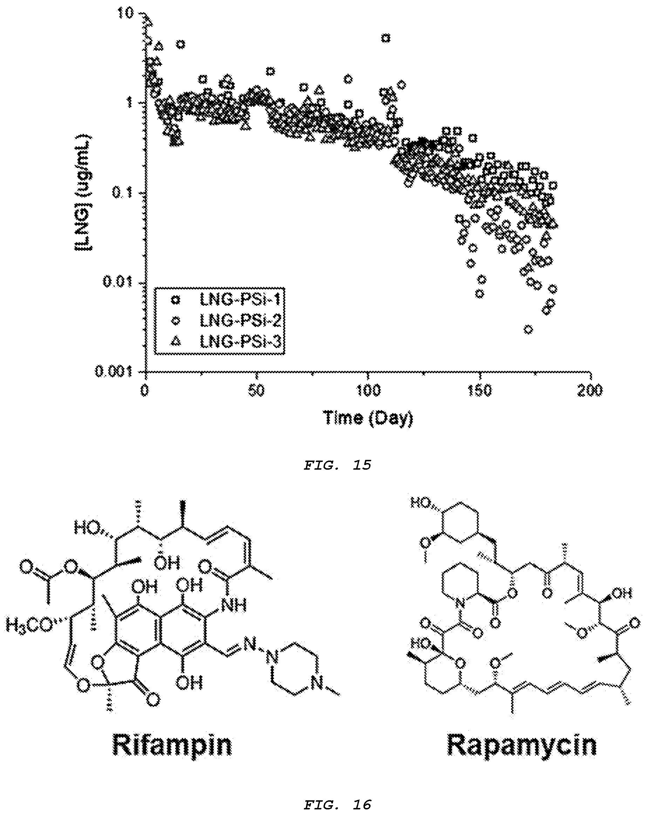

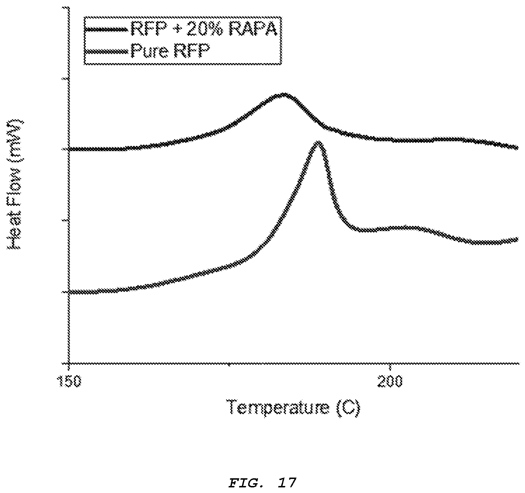

In addition to contraceptive therapeutic agents, additional families of drugs were investigated for their viability for melt casting. One such drug included rifampin (RFP), a rifamycin family molecule used as a potent antibiotic. Similar to levonorgestrel, pure RFP could not be melt cast into a porous silicon material due to its thermal instability. Specifically, it was found that RFP began melting at approximately 178 °C but began losing mass from volatile degradation products at 184 °C. To broaden the window, a similarly structured molecule, rapamycin (RAPA), was mechanically mixed with RFP at a ratio of 4:1(RFP:RAPA) (*e.g*., see **FIG. 16**). It was found that the incorporation of RAPA significantly depressed and broadened the temperature range of RFP melting, thus lowering and broadening the window of thermal stability for melt casting RFP into porous silicon materials disclosed herein (*e.g.,* see **FIG. 17**). The later finding indicates that clear synergistic effects can be achieved by use of two or more therapeutic drugs in combination not only for use in treating a specific disorder but in melt casting the drug products into porous silicon materials.

It was further found herein that the release profile of the drug product can be tuned by controlling the physiochemical properties of the porous silicon materials of the disclosure. Key preparative parameters for the porous silicon materials were systematically modulated in order to determine their effect on temporal drug release profiles. The mass loading of the porous silicon materials of the disclosure with segesterone acetate was found to have a substantial effect on particle dissolution rate. The ratio of drug-to-host substantially modifies the relative hydrophobic character of the particle surface, and this apparently determines (in part) the rate of drug release. Thus, samples with 20% (by mass) segesterone acetate loading released all drug in approximately 60 days, while formulations with 70% segesterone acetate required 120 days to dissolve. It should be noted that even the shortest dissolution period for segesterone acetate-loaded particles exceeded the time required for pure segesterone acetate to dissolve under the same test conditions. Thus, drug loading provides a means to tune dosing and duration of action. The size of the pores in the porous silicon material was found to have the largest impact on the rate of segesterone acetate dissolution. Pore morphology is primarily dictated by the process used to prepare the porous silicon material. In addition to having more void space, a higher porosity particle also has a larger pore diameter and lower particle density. It was found that porosity exerted a strong effect on the temporal drug release profiles. For example, 35% porosity particles released segesterone acetate into solution as rapidly as crystalline segesterone acetate controls (40 days). Higher porosity samples showed larger pore openings, much greater drug loading, and substantially longer release periods; porous silica particles with 75% porosity yielded extended release for > 3 months, with a highly linear release (constant steady-state drug concentration *in vitro*)*,* and with a rapid drop-off at the end of the release period. The preparation method was found to be reproducible from batch to batch; mass loading for five unique batches was determined by thermogravimetric analysis to be 70.6% with a standard deviation of 2.3%. Temporal drug release profiles for all five batches were reproducible within a tolerance of 17.3%.

The porous silicon materials, including porous silicon particles, disclosed herein were also well tolerated *in vivo* when loaded with segesterone acetate. Adult female Sprague-Dawley rats (approx. 300 g) were used to assess the tolerability of segesterone acetate-loaded porous silicon materials. After 6 months, animals were sacrificed and tissues were collected, fixed, embedded, sectioned, and stained for hematoxylin and eosin. Tissues collected included the heart, brain, lungs, liver, kidneys, spleen, and ovaries (with fallopian tubes). All tissues examined in all groups were graded as normal. The animals showed no adverse reaction to either empty porous silicon particles or to segesterone acetate-loaded porous silicon particles. Serum samples were collected at the 6-month point and analyzed for segesterone acetate via HPLC-MS/MS techniques. Segesterone acetate was detected in the serum of all NES-containing groups, including the segesterone acetate group without porous silicon particles.

Reproducible sustained delivery of a drug at a target site is one of the main themes in controlled drug-delivery systems. The most commonly used drug-delivery systems, which can release drugs longer than one week, are parenteral injections and implants. Certain implant systems can deliver drugs for more than one year, and the longest drug delivery can be achieved by biodegradable or non-biodegradable implant systems. Long-acting injectable formulations offer many advantages when compared with conventional formulations of the same compounds. These advantages include the following: a predictable drug-release profile during a defined period of time following each injection; better patient compliance; ease of application; improved systemic availability by avoidance of first-pass metabolism; reduced dosing frequency (*i.e.,* fewer injections) without compromising the effectiveness of the treatment; decreased incidence of side effects; and overall cost reduction of medical care.

As shown in the studies presented herein, porous silicon particles that have been infiltrated with one or more thermally unstable therapeutic drugs using a melt casting approach provide for extended and steady state delivery of therapeutic drugs for many weeks. As such, the compositions disclosed herein are ideally suited for use as depot formulations for the long term controlled delivery of therapeutic agents. For example, the compositions disclosed herein provide for controlled delivery of therapeutic agents up to 30 days, 40 days, 50 days, 60 days, 70 days, 80 days, 90 days, 100 days, 120 days, 130 days, 140 days, 150 days, 160 days, 170 days, 180 days, 190 days, 200 days, or any range of time periods that includes or is between any two of the foregoing time points.

In further embodiment, the disclosure provides for pharmaceutical compositions which comprise the drug delivery formulations disclosed herein. The pharmaceutical compositions disclosed herein may be administered parenterally by injection, infusion, or implantation, for local or systemic administration. Parenteral administration, as used herein, includes intravenous, intraarterial, intraperitoneal, intrathecal, intraventricular, intraurethral, intrasternal, intracranial, intramuscular, intrasynovial, and subcutaneous administration.

The pharmaceutical compositions disclosed herein may be formulated in any dosage forms that are suitable for parenteral administration, including solutions, suspensions, emulsions, and solid forms suitable for solutions or suspensions in liquid prior to injection. Such dosage forms can be prepared according to conventional methods known to those skilled in the art of pharmaceutical science (see, *Remington: The Science and Practice of Pharmacy,* supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (e.g., polyethylene glycol 300 and polyethylene glycol 400), propylene glycol, glycerin, N-methyl-2-pyrrolidone, dimethylacetamide, and dimethylsulfoxide.

In one embodiment, the pharmaceutical compositions disclosed herein are formulated as ready-to-use sterile solutions. In another embodiment, the pharmaceutical compositions are disclosed herein are formulated as sterile dry soluble products, including powders and hypodermic tablets, which, if so necessary, may be reconstituted with a vehicle prior to use. In yet another embodiment, the pharmaceutical compositions are disclosed as ready-to-use sterile suspensions or emulsions. In yet another embodiment, the pharmaceutical compositions are disclosed as sterile dry insoluble products to be reconstituted with a vehicle prior to use.

The pharmaceutical compositions may be formulated as a suspension, solid, semi-solid, or thixotropic liquid, for administration as an implanted depot.

The pharmaceutical compositions disclosed herein may be administered intranasally or by inhalation to the respiratory tract. The pharmaceutical compositions may be disclosed in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be disclosed as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient disclosed herein, a propellant as solvent; and/or an surfactant, such as sorbitan trioleate, oleic acid, or an oligolactic acid.

The pharmaceutical compositions disclosed herein may be micronized to a size suitable for delivery by inhalation, such as 10 micrometers or less. Particles of such sizes may be prepared using a comminuting method known to those skilled in the art, such as spiral jet milling, fluid bed jet milling, and supercritical fluid processing to form nanoparticles, high pressure homogenization, or spray drying.

Capsules, blisters and cartridges for use in an inhaler or insufflator may be formulated to contain a powder mix of the pharmaceutical compositions disclosed herein; a suitable powder base, such as lactose or starch; and a performance modifier, such as l-leucine, mannitol, or magnesium stearate. The lactose may be anhydrous or in the form of the monohydrate. Other suitable excipients or carriers include dextran, glucose, maltose, sorbitol, xylitol, fructose, sucrose, and trehalose. The pharmaceutical compositions disclosed herein for inhaled/intranasal administration may further comprise a suitable flavor, such as menthol and levomenthol, or sweeteners, such as saccharin or saccharin sodium.

In a particular embodiment, the porous silicon materials disclosed herein can be infiltrated with one or more drugs or therapeutic agents (*e.g.,* small molecular drugs and the like, biological agents and protein/peptide drugs). Examples of therapeutic agents, include but are not limited, hormone therapy agents (*e.g.,* somatropin, testosterone, estrogen); contraceptive agents (*e.g.,* progestins, progestins and estrogen); protein therapeutics (*e.g.,* analog of glucagon-likepeptide-1); recombinant human bone morphogenetic protein-2; superoxide dismutase; calcitonin; insulin; gene delivery (*e.g.,* plasmid DNA); cancer therapeutic agents (*e.g.,* bleomycin, paclitaxel, cisplatin); therapeutic antibodies (*e.g.,* abciximab, ramucirumab); peptide-like antineoplastic agents; postoperative pain therapeutic agents (*e.g.,* Ketorolactromethamine); schizophrenia drugs (*e.g.,* aripiprazole, olanzapine); peptide vaccines; drugs to treat alcohol dependence (*e.g.,* Naltrexone); and immunosuppressive drugs (*e.g.,* Rapamycin). In a further embodiment, the porous silicon materials disclosed herein can be infiltrated with one or more melting point suppression agents. The drugs or therapeutic agents of the instant disclosure typically undergo a phase transition from a solid to a liquid (*i.e.,* melt) upon the application of heat. Further application of heat results in the decomposition of the drug or therapeutic agent. The melting temperature and decomposition temperature of the drug or therapeutic agent is largely dependent on the structure of the particular drug or therapeutic agent. For drugs or therapeutic agents where the difference in temperature between the melting temperature and the decomposition temperature is small, it was found that the use of a meltable composition comprising the drug or therapeutic agent and a melting point suppression agent could provide the beneficial effect of expanding the window between the point in which the drug or therapeutic agent melts and when it decomposes.

Without intending to be bound by theory, it is thought that the mixture of a drug or therapeutic agent and a melting point suppression agent may disrupt the close packing of the drug or therapeutic agent, thereby decreasing the amount/number of stabilizing interactions between molecules of the drug or therapeutic agent. In other words, less applied heat is necessary to the thermally unstable substance by forming less stabilization interactions with the thermally unstable drug or therapeutic agent causes the thermally unstable drug or therapeutic agent to undergo a phase transition to liquid form using less applied heat. In order to increase 'favorable' interactions between the thermally unstable substance with the thermally unstable drug or therapeutic agent, the thermally unstable substance is typically structurally similar to the thermally unstable drug or therapeutic agent. It has been advantageously found herein, that the thermally unstable compound can be a second thermally unstable drug or therapeutic agent. Thus, synergies between increased efficacy by using combined therapies and being able to load the porous silicon materials with a thermally unstable drug or agent that has a narrow window between its melting and degradation temperature can be realized by loading the same porous silicon materials with a second thermally unstable drug or agent, which itself may also have a narrow window between its melting and degradation temperature. Such combined therapy option can be especially beneficial for treating a disease or disorder where there is a clear benefit by administering multiple drugs or therapeutics agents, *e*.g., cancer (*e.g.,* multiple chemotherapeutics), heart disease (*e.g.,* antihypertensive with a diuretic), pain (*e.g.,* multiple pain relievers), contraception (*e.g.,* estrogen with a progestin or progesterone), infections (*e.g.,* multiple antibiotics), etc.

The drug delivery formulations disclosed herein may also be combined or used in combination with other agents useful in the treatment, prevention, or amelioration of one or more symptoms of disease or disorder. Or, by way of example only, the therapeutic effectiveness of the drug delivery formulations disclosed herein may be enhanced by administration of an adjuvant (*i.e.,* by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used, simultaneously or sequentially with a drug delivery formulation disclosed herein. When a drug delivery formulation disclosed herein is used contemporaneously with one or more other drugs, a pharmaceutical composition containing such other drugs in addition to the drug delivery formulation disclosed herein may be utilized, but is not required. As indicated above, the drug delivery formulations of the disclosure can include multiple therapeutic agents. Thus, the drug delivery formulations of the disclosure can provide for the extended and simultaneous delivery of multiple drug products.

For example, in certain embodiments, the drug delivery formulations disclosed herein can be used to deliver or be combined with one or more diuretics, one or more adrenergic receptor antagonists, one or more HMG-CoA reductase inhibitors, one or more diabetes mellitus treatments, one or more steroidal drugs, one or more antibacterial agents, one or more antifungal agents, one or more anticoagulants, one or more thrombolytics, one or more non-steroidal anti-inflammatory agents, one or more antiplatelet agents, one or more anti-cancer agents, one or more anti-convulsant (anti-seizure) agents, one or more anti-depressant agents, one or more anti-hypertensive agents, one or more cardiac agents, one or more cardiovascular agents, one or more CNS and respiratory stimulants, one or more hypnotic agents or sedatives, one or more muscarinic receptor agonists, one or more neuroleptic agents, one or more peptide drugs, or one or more sex steroids, including any of the sex steroids listed herein.

Exemplary diuretics include, but are not limited to, bendroflumethiazide, hydroflumethiazide, hydrochlorothiazide, chlorothiazide, polythiazide, trichlormethiazide, cyclopenthiazide, methyclothiazide, cyclothiazide, mebutizide, quinethazone, clopamide, chlortalidone, mefruside, clofenamide, metolazone, meticrane, xipamide, indapamide, clorexolone, fenquizone, mersalyl, theobromine, cicletanine, furosemide, bumetanide, piretanide, torasemide, etacrynic acid, tienilic acid, muzolimine, etozolin, spironolactone, potassium canrenoate, canrenone, and eplerenone. Exemplary adrenergic receptor antagonists inlcude, but are not limited to, propranolol, atenolol, metoprolol, nadolol, oxprenolol, pindolol, propranolol, timolol, doxazosin, phentolamine, indoramin, phenoxybenzamine, prazosin, terazosin, tolazoline, bucindolol, carvedilol, and labetalol. Exemplary HMG-CoA reductase inhibitors include, but are not limited to, atorvastatin, cerivastatin, fluvastatin, lovastatin, mevastatin, pitavastatin, pravastatin, rosuvastatin, and simvastatin. Exemplary drugs for treating diabetes include, but are not limited to, insulin (human, beef, pork, lispro, aspart, glulisine, glargine, or detemir), phenformin, metformin, buformin, glibenclamide, chlorpropamide, tolbutamide, glibornuride, tolazamide, carbutamide, glipizide, gliquidone, gliclazide, metahexamide, glisoxepide, glimepiride, acetohexamide, glymidine, acarbose, miglitol, voglibose, troglitazone, rosiglitazone, pioglitazone, sitagliptin, vildagliptin, guar gum, repaglinide, nateglinide and exenatide. Exemplary steroidal drugs include, but are not limited to, aldosterone, beclometasone, betamethasone, deoxycorticosterone acetate, fludrocortisone acetate, hydrocortisone (cortisol), prednisolone, prednisone, methylprenisolone, dexamethasone, and triamcinolone. Exemplary antibacterial agents, include, but are not limited to amikacin, amoxicillin, ampicillin, arsphenamine, azithromycin, aztreonam, azlocillin, bacitracin, carbenicillin, cefaclor, cefadroxil, cefamandole, cefazolin, cephalexin, cefdinir, cefditorin, cefepime, cefixime, cefoperazone, cefotaxime, cefoxitin, cefpodoxime, cefprozil, ceftazidime, ceftibuten, ceftizoxime, ceftriaxone, cefuroxime, chloramphenicol, cilastin, ciprofloxacin, clarithromycin, clindamycin, cloxacillin, colistin, dalfopristan, demeclocycline, dicloxacillin, dirithromycin, doxycycline, erythromycin, enafloxacin, ertepenem, ethambutol, flucloxacillin, fosfomycin, furazolidone, gatifloxacin, geldanamycin, gentamicin, herbimicin, imipenem, isoniazide, kanamicin, levofloxacin, linezolid, lomefloxacin, loracarbef, mafenide, moxifloxacin, meropenem, metronidazole, mezlocillin, minocycline, mupirozin, nafcillin, neomycin, netilmicin, nitrofurantoin, norfloxacin, ofloxacin, oxytetracycline, penicillin, piperacillin, platensimycin, polymixin B, prontocil, pyrazinamide, quinupristine, rifampin, roxithromycin, spectinomycin, streptomycin, sulfacetamide, sulfamethizole, sulfamethoxazole, teicoplanin, telithromycin, tetracycline, ticarcillin, tobramycin, trimethoprim, troleandomycin, trovafloxacin, and vancomycin. Exemplary antifungal agents include, but are not limited to, amorolfine, amphotericin B, anidulafungin, bifonazole, butenafine, butoconazole, caspofungin, ciclopirox, clotrimazole, econazole, fenticonazole, filipin, fluconazole, isoconazole, itraconazole, ketoconazole, micafungin, miconazole, naftifine, natamycin, nystatin, oxyconazole, ravuconazole, posaconazole, rimocidin, sertaconazole, sulconazole, terbinafine, terconazole, tioconazole, and voriconazole. Exemplary anticoagulants include, but are not limited to, acenocoumarol, argatroban, bivalirudin, lepirudin, fondaparinux, heparin, phenindione, warfarin, and ximalagatran. Exemplary thrombolytics include, but are not limited to, anistreplase, reteplase, t-PA (alteplase activase), streptokinase, tenecteplase, and urokinase. Exemplary non-steroidal anti-inflammatory agents include, but are not limited to, aceclofenac, acemetacin, amoxiprin, aspirin, azapropazone, benorilate, bromfenac, carprofen, celecoxib, choline magnesium salicylate, diclofenac, diflunisal, etodolac, etoracoxib, faislamine, fenbuten, fenoprofen, flurbiprofen, ibuprofen, indometacin, ketoprofen, ketorolac, lomoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinprazone, suprofen, tenoxicam, tiaprofenic acid, and tolmetin. Exemplary antiplatelet agents include, but are not limited to, abciximab, cilostazol, clopidogrel, dipyridamole, ticlopidine, and tirofibin. Exemplary antineoplastic agents include, but are not limited to, paclitaxel, docetaxel, camptothecin and its analogues and derivatives (e.g., 9-aminocamptothecin, 9-nitrocamptothecin, 10-hydroxy-camptothecin, irinotecan, topotecan, 20-O-β-glucopyranosyl camptothecin), taxanes (baccatins, cephalomannine and their derivatives), carboplatin, cisplatin, interferon-α_{2A}, interferon-α_{2B}, interferon-α_{N3} and other agents of the interferon family, levamisole, altretamine, cladribine, tretinoin, procarbazine, dacarbazine, gemcitabine, mitotane, asparaginase, porfimer, mesna, amifostine, mitotic inhibitors including podophyllotoxin derivatives such as teniposide and etoposide and vinca alkaloids such as vinorelbine, vincristine and vinblastine. Exemplary anti-convulsant (anti-seizure) agents include, but are not limited to, azetazolamide, carbamazepine, clonazepam, clorazepate, ethosuximide, ethotoin, felbamate, lamotrigine, mephenyloin, mephobarbital, phenyloin, phenobarbital, primidone, trimethadione, vigabatrin, topiramate, and the benzodiazepines. Benzodiazepines, as is well known, are useful for a number of indications, including anxiety, insomnia, and nausea. Examples of suitable commercially available anti-convulsants useful in the dosage forms of include TEGRETOL^{®} (carbamazepine; Novartis, Summit, N.J.), DILANTIN^{®} (Pfizer Inc., New York, N.Y.) and LAMICTAL^{®} (lamotrigine (GlaxoSmithKline, Philadelphia, Pa.). Exemplary anti-depressant agents include, but are not limited to, tricyclic antidepressants LIMBITROL^{®} (amitriptyline; Hoffmann-LaRoche, Nutley, N.J.), TOFRANIL^{®} (imipramine; Tyco Healthcare, Mansfiled, Mass.), ANAFRANIL^{™} (clomipramine; Tyco Healthcare, Mansfield, Mass.), and NORPRAMIN^{®} (desipramine; Sanofi-Aventis, Bridgewater, N.J.). Exemplary anti-hypertensive agents include, but are not limited to, amlodipine, benazepril, darodipine, diltiazem, doxazosin, enalapril, eposartan, esmolol, felodipine, fenoldopam, fosinopril, guanabenz, guanadrel, guanethidine, guanfacine, hydralazine, losartan, metyrosine, minoxidil, nicardipine, nifedipine, nisoldipine, phenoxybenzamine, prazosin, quinapril, reserpine, terazosin, and valsartan. Exemplary cardiac agents, include, but are not limited to, amiodarone, amlodipine, atenolol, bepridil, bisoprolol bretylium, captopril, carvedilol, diltiazem, disopyramide, dofetilide, enalaprilat, enalapril, encamide, esmolol, flecamide, fosinopril, ibutilide, inaminone, irbesartan, lidocaine, lisinopril, losartan, metroprolol, nadolol, nicardipine, nifedipine, procainamide, propafenone, propranolol, quinapril, quinidine, ramipril, trandolapril, and verapamil. Exemplary cardiovascular agents include, but are not limited to, angiotensin converting enzyme (ACE) inhibitors, cardiac glycosides, calcium channel blockers, beta-blockers, antiarrhythmics, cardioprotective agents, and angiotensin II receptor blocking agents. Examples of the foregoing classes of drugs include the following: ACE inhibitors such as enalapril, 1-carboxymethyl-3-1-carboxy-3-phenyl-(1S)-propylamino-2,3,4,5-tetrahydro-1H-(3S)-1 benzazepine-2-one, 3-(5-amino-1-carboxy-1S-pentyl)amino-2,3,4,5-tetrahydro-2-oxo-3S-1H-1-benzazepine-1-acetic acid or 3-(1-ethoxycarbonyl-3-phenyl-(1S)-propylamino)-2,3,4,5-tetrahydro-2-oxo-(3S)-benzazepine-1-acetic acid monohydrochloride; cardiac glycosides such as digoxin and digitoxin; inotropes such as aminone and milrinone; calcium channel blockers such as verapamil, nifedipine, nicardipene, felodipine, isradipine, nimodipine, bepridil, amlodipine and diltiazem; beta-blockers such as atenolol, metoprolol; pindolol, propafenone, propranolol, esmolol, sotalol, timolol, and acebutolol; antiarrhythmics such as moricizine, ibutilide, procainamide, quinidine, disopyramide, lidocaine, phenyloin, tocamide, mexiletine, flecamide, encamide, bretylium and amiodarone; and cardioprotective agents such as dexrazoxane and leucovorin; vasodilators such as nitroglycerin; and angiotensin II receptor blocking agents such as losartan, hydrochlorothiazide, irbesartan, candesartan, telmisartan, eposartan, and valsartan. Exemplary CNS and respiratory stimulants include, but are not limited to, xanthines such as caffeine and theophylline; amphetamines such as amphetamine, benzphetamine hydrochloride, dextroamphetamine, dextroamphetamine sulfate, levamphetamine, levamphetamine hydrochloride, methamphetamine, and methamphetamine hydrochloride; and miscellaneous stimulants such as methylphenidate, methylphenidate hydrochloride, modafinil, pemoline, sibutramine, and sibutramine hydrochloride. Exemplary hypnotic agents and sedatives include, but are not limited to, clomethiazole, ethinamate, etomidate, glutethimide, meprobamate, methyprylon, zolpidem, and barbiturates (*e.g.,* amobarbital, apropbarbital, butabarbital, butalbital, mephobarbital, methohexital, pentobarbital, phenobarbital, secobarbital, thiopental). Exemplary muscarinic receptor agonists include, but are not limited to, choline esters such as acetylcholine, methacholine, carbachol, bethanechol (carbamylmethylcholine), bethanechol chloride, cholinomimetic natural alkaloids and synthetic analogs thereof, including pilocarpine, muscarine, McN-A-343, and oxotremorine. Muscarinic receptor antagonists are generally belladonna alkaloids or semisynthetic or synthetic analogs thereof, such as atropine, scopolamine, homatropine, homatropine methyl bromide, ipratropium, methantheline, methscopolamine and tiotropium. Exemplary neuroleptic agents, include, but are not limited to, antidepressant drugs, antimanic drugs, and antipsychotic agents, wherein antidepressant drugs include (a) the tricyclic antidepressants such as amoxapine, amitriptyline, clomipramine, desipramine, doxepin, imipramine, maprotiline, nortriptyline, protriptyline, and trimipramine, (b) the serotonin reuptake inhibitors citalopram, fluoxetine, fluvoxamine, paroxetine, sertraline, and venlafaxine, (c) monoamine oxidase inhibitors such as phenelzine, tranylcypromine, and (-)-selegiline, and (d) other, "atypical" antidepressants such as nefazodone, trazodone and venlafaxine, and wherein antimanic and antipsychotic agents include (a) phenothiazines such as acetophenazine, acetophenazine maleate, chlorpromazine, chiorpromazine hydrochloride, fluphenazine, fluphenazine hydrochloride, fluphenazine enanthate, fluphenazine decanoate, mesoridazine, mesoridazine besylate, perphenazine, thioridazine, thioridazine hydrochloride, trifluoperazine, and trifluoperazine hydrochloride, (b) thioxanthenes such as chlorprothixene, thiothixene, and thiothixene hydrochloride, and (c) other heterocyclic drugs such as carbamazepine, clozapine, droperidol, haloperidol, haloperidol decanoate, loxapine succinate, molindone, molindone hydrochloride, olanzapine, pimozide, quetiapine, risperidone, and sertindole. Exemplary peptide drugs include, but are not limited to, peptidyl hormones activin, amylin, angiotensin, atrial natriuretic peptide (ANP), calcitonin, calcitonin gene-related peptide, calcitonin N-terminal flanking peptide, ciliary neurotrophic factor (CNTF), corticotropin (adrenocorticotropin hormone, ACTH), corticotropin-releasing factor (CRF or CRH), epidermal growth factor (EGF), follicle-stimulating hormone (FSH), gastrin, gastrin inhibitory peptide (GIP), gastrin-releasing peptide, gonadotropin-releasing factor (GnRF or GNRH), growth hormone releasing factor (GRF, GRH), human chorionic gonadotropin (hCH), inhibin A, inhibin B, insulin, luteinizing hormone (LH), luteinizing hormone-releasing hormone (LHRH), α-melanocyte-stimulating hormone, β-melanocyte-stimulating hormone, γ-melanocyte-stimulating hormone, melatonin, motilin, oxytocin (pitocin), pancreatic polypeptide, parathyroid hormone (PTH), placental lactogen, prolactin (PRL), prolactin-release inhibiting factor (PI F), prolactin-releasing factor (PRF), secretin, somatotropin (growth hormone, GH), somatostatin (SIF, growth hormone-release inhibiting factor, GIF), thyrotropin (thyroid-stimulating hormone, TSH), thyrotropin-releasing factor (TRH or TRF), thyroxine, vasoactive intestinal peptide (VIP), and vasopressin. Other peptidyl drugs are the cytokines, *e.g.,* colony stimulating factor 4, heparin binding neurotrophic factor (HBNF), interferon-α, interferon α-2a, interferon α-2b, interferon α-n3, interferon-β, etc., interleukin-1, interleukin-2, interleukin-3, interleukin-4, interleukin-5, interieukin-6, etc., tumor necrosis factor, tumor necrosis factor-a, granuloycte colony-stimulating factor (G-CSF), granulocyte-macrophage colony-stimulating factor (GM-CSF), macrophage colony-stimulating factor, midkine (MD), and thymopoietin. Still other peptidyl drugs that can be advantageously delivered using the present systems include endorphins (*e.g.,* dermorphin, dynorphin, α-endorphin, α-endorphin, γ-endorphin, α-endorphin, [Leu⁵]enkephalin, [Met⁵]enkephalin, substance P), kinins (*e.g.,* bradykinin, potentiator B, bradykinin potentiator C, kallidin), LHRH analogues (*e.g.,* buserelin, deslorelin, fertirelin, goserelin, histrelin, leuprolide, lutrelin, nafarelin, tryptorelin), and the coagulation factors, such as α₁-antitrypsin, α₂-macroglobulin, antithrombin III, factor I (fibrinogen), factor II (prothrombin), factor III (tissue prothrombin), factor V (proaccelerin), factor VII (proconvertin), factor VII (antihemophilic globulin or AHG), factor IX (Christmas factor, plasma thromboplastin component or PTC), factor X (Stuart-Power factor), factor XI (plasma thromboplastin antecedent or PTA), factor XII (Hageman factor), heparin cofactor II, kallikrein, plasmin, plasminogen, prekallikrein, protein C, protein S, and thrombomodulin and combinations thereof. Exemplary sex steroids include, but are not limited to, progestogens or progestins, such as acetoxypregnenolone, allylestrenol, anagestone acetate, chlormadinone acetate, cyproterone, cyproterone acetate, desogestrel, dihydrogesterone, dimethisterone, ethisterone (17α-ethinyltestosterone), ethynodiol diacetate, fluorogestone acetate, gestadene, hydroxyprogesterone, hydroxyprogesterone acetate, hydroxyprogesterone caproate, hydroxymethylprogesterone, hydroxymethylprogesterone acetate, 3-ketodesogestrel, levonorgestrel, lynestrenol, medrogestone, medroxyprogesterone acetate, megestrol, megestrol acetate, melengestrol acetate, norethindrone, norethindrone acetate, norethisterone, norethisterone acetate, norethynodrel, norgestimate, norgestrel, norgestrienone, normethisterone, and progesterone. Also included within this general class are estrogens, *e.g*.: estradiol (*i.e.,* 1,3,5-estratriene-3,17β-diol, or "17β-estradiol") and its esters, including estradiol benzoate, valerate, cypionate, heptanoate, decanoate, acetate and diacetate; 17α-estradiol; ethinylestradiol (*i.e.,* 17α-ethinylestradiol) and esters and ethers thereof, including ethinylestradiol 3-acetate and ethinylestradiol 3-benzoate; estriol and estriol succinate; polyestrol phosphate; estrone and its esters and derivatives, including estrone acetate, estrone sulfate, and piperazine estrone sulfate; quinestrol; mestranol; and conjugated equine estrogens. Androgenic agents, also included within the general class of sex steroids, are drugs such as the naturally occurring androgens androsterone, androsterone acetate, androsterone propionate, androsterone benzoate, a ndrostenediol, androstenediol-3-acetate, and rostenediol-17-acetate, androstenediol-3,17-diacetate, androstenediol 17-benzoate, androstenediol-3-acetate-17-benzoate, androstenedione, dehydroepiandrosterone (DHEA; also termed "prasterone"), sodium dehydroepiandrosterone sulfate, 4-dihydrotestosterone (DHT; also termed "stanolone"), 5α-dihydrotestosterone, dromostanolone, dromostanolone propionate, ethylestrenol, nandrolone phenpropionate, nandrolone decanoate, nandrolone furylpropionate, nandrolone cyclohexanepropionate, nandrolone benzoate, nandrolone cyclohexanecarboxylate, oxandrolone, stanozolol and testosterone; pharmaceutically acceptable esters of testosterone and 4-dihydrotestosterone, typically esters formed from the hydroxyl group present at the C-17 position, including, but not limited to, the enanthate, propionate, cypionate, phenylacetate, acetate, isobutyrate, buciclate, heptanoate, decanoate, undecanoate, caprate and isocaprate esters; and pharmaceutically acceptable derivatives of testosterone such as methyl testosterone, testolactone, oxymetholone and fluoxymesterone.

In another embodiment, the drug delivery formulations and compositions disclosed herein can be used to deliver or be combined with one or more classes of drugs, including, but not limited to, endothelin converting enzyme (ECE) inhibitors, such as phosphoramidon; thromboxane receptor antagonists, such as ifetroban; potassium channel openers; thrombin inhibitors, such as hirudin; growth factor inhibitors, such as modulators of PDGF activity; platelet activating factor (PAF) antagonists; anti-platelet agents, such as GPIIb/IIIa blockers (*e.g.,* abdximab, eptifibatide, and tirofiban), P2Y(AC) antagonists (*e.g.,* clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-PPAR-γ and/or angiotensin II agents; beta-PPAR-γ and/or angiotensin II agents, such as carvedilol and metoprolol; antiarrhythmic agents; diuretics, such as chlorothlazide, hydrochlorothiazide, flumethiazide, hydroflumethiazide, bendroflumethiazide, methylchlorothiazide, trichloromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prourokinase, and anisoylated plasminogen streptokinase activator complex (APSAC); anti-diabetic agents, such as biguanides (*e.g.* metformin), glucosidase inhibitors (*e.g.,* acarbose), insulins, meglitinides (*e.g.,* repaglinide), sulfonylureas (*e.g.,* glimepiride, glyburide, and glipizide), thiozolidinediones (*e.g.* troglitazone, rosiglitazone and pioglitazone), and PPAR-gamma agonists; mineralocorticoid receptor antagonists, such as spironolactone and eplerenone; growth hormone secretagogues; aP2 inhibitors; phosphodiesterase inhibitors, such as PDE III inhibitors (*e.g.,* cilostazol) and PDE V inhibitors (*e.g.,* sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (*e.g.,* alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (*e.g.,* cortisone), estrogens/antiestrogens, androgens/antiandrogens, progestins, and luteinizing hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

Where appropriate, any of the therapeutic agents or drugs described herein may be provided in the form of a salt, ester, amide, prodrug, conjugate, active metabolite, isomer, fragment, analog, or the like, provided that the salt, ester, amide, prodrug, conjugate, active metabolite, isomer, fragment, or analog is pharmaceutically acceptable and pharmacologically active in the present context. Salts, esters, amides, prodrugs, conjugates, active metabolites, isomers, fragments, and analogs of the agents may be prepared using standard procedures known to those skilled in the art of synthetic organic chemistry and described, for example, by J. March, Advanced Organic Chemisty: Reactions, Mechanisms and Structure, 5th Edition (New York: Wiley-Interscience, 2001). For example, where appropriate, any of the therapeutic agents or drugs described herein may be in the form of a prodrug. The prodrug requires conversion to the active agent.

The disclosure provides in certain embodiments a drug delivery composition comprising: a porous silicon material having a porosity from about 15% to about 85%, wherein the pores of the silicon material are loaded with a mixture comprising a thermally unstable therapeutic agent and a thermally unstable substance, wherein the thermally unstable therapeutic agent and the thermally unstable substance are not the same compound or molecule, and wherein the mixture has a lower melting point than the melting point of the thermally unstable therapeutic agent. In another embodiment, the porous silicon material disclosed herein are porous silicon particles. In yet another embodiment, the porous silicon particles of the disclosure have an average dimeter or length from about 10 nm to about 100 µm. In a further embodiment, the porous silicon particles of the disclosure have an average dimeter or length from about 10 nm to about 100 nm. In an alternate embodiment, the porous silicon particles have an average dimeter or length from about 100 nm to about 100 µm. In a certain embodiment, the porous silicon material of the disclosure has a porosity from about 50% to about 80%. In another embodiment, the porous silicon material of the disclosure has a porosity of about 75%. In yet another embodiment, the porous silicon material of the disclosure comprises pores that have average diameters from about 2 nm to about 250 nm. In a further embodiment, the porous silicon material of the disclosure comprises pores that have average diameters from about 20 nm to about 150 nm. In yet another embodiment, the difference in temperature from which the thermally unstable therapeutic agent begins to melt to when the thermally unstable therapeutic agent begins to degrade is less than about 10 °C. In a further embodiment, the difference in temperature from which the thermally unstable therapeutic agent begins to melt to when the thermally unstable therapeutic agent begins to degrade is less than about 5 °C. In yet a further embodiment, the difference in temperature from which the thermally unstable therapeutic agent begins to melt to when the thermally unstable therapeutic agent begins to degrade is from about 2 °C to about 3 °C. In a certain embodiment, the melting point of the mixture is at least 10 °C lower than the melting point of the thermally unstable therapeutic agent. In another embodiment, the melting point of the mixture is at least 20 °C lower than the melting point of the thermally unstable therapeutic agent. In yet another embodiment, the melting point of the mixture is at least 30 °C lower than the melting point of the thermally unstable therapeutic agent. In a further embodiment, the mixture is a eutectic mixture. In yet a further embodiment, the mixture has a ratio of thermally unstable therapeutic agent to a thermally unstable substance from 1:10 to 10:1. In a particular embodiment, the mixture has a ratio of thermally unstable therapeutic agent to a thermally unstable substance from 1:5 to 5:1. In another embodiment, the mixture has a ratio of thermally unstable therapeutic agent to a thermally unstable substance from 1:3 to 3:1. In yet another embodiment, the thermally unstable substance has a structure that is highly similar to the thermally unstable therapeutic agent. According to the invention, the thermally unstable therapeutic agent is a contraceptive agent. In a further embodiment, the contraceptive agent is a progestin or progesterone. In another embodiment, the thermally unstable substance is cholesterol. In an alternate embodiment, the thermally unstable substance is a second thermally unstable therapeutic agent. In another embodiment, the thermally unstable therapeutic agent and second unstable therapeutic agent are of the same drug class. In an alternate embodiment, the thermally unstable therapeutic agent and second unstable therapeutic agent have therapeutic effectiveness in treating the same medical condition or disorder in a subject. In a further embodiment, the medical condition or disorder is selected from heart disease, diabetes, attention deficit hyperactivity disorder (ADHD), schizophrenia, anxiety, pain, bacterial infections, viral infections, protozoan infections, cancer, motion sickness, hypertension, and symptoms associated with post-menopause. In another embodiment, the thermally unstable therapeutic agent is a progestin or progesterone and the second unstable therapeutic agent is estrogen or a second progestin. In yet another embodiment, the porous silicon material has been thermally oxidized at temperature of 800 °C or greater for 1 hour or greater. In a further embodiment, the drug delivery system of the disclosure is capable of releasing the thermally unstable therapeutic agent *in vivo* or *in vitro* in a linear and sustained manner for at least 30 days. In yet a further embodiment, the drug delivery system of the disclosure is capable of releasing the thermally unstable therapeutic agent *in vivo* or *in vitro* in a linear and sustained manner for at least 60 days. In a certain embodiment, the drug delivery composition of the disclosure is capable of releasing the thermally unstable therapeutic agent *in vivo* or *in vitro* in a linear and sustained manner for at least 100 days.

In a particular embodiment, the disclosure also provides for a method to make a drug delivery composition of the disclosure that comprises porous silicon material(s) that have been melt-casted with a thermally unstable therapeutic agent or a mixture thereof, comprising heating a dry mixture comprising: (a) porous silicon material(s), and (b) an unstable therapeutic agent, or (c) a loading mixture comprising an unstable therapeutic agent and a thermally unstable substance, under an inert atmosphere at a temperature sufficient for melting (b) the unstable therapeutic agent or (c) the mixture comprising the unstable therapeutic agent and a thermally unstable substance, wherein the temperature is not sufficient to cause degradation of the unstable therapeutic agent of (b) or (c); maintaining the porous silicon material(s) with (b) the unstable therapeutic agent or (c) the mixture comprising the unstable therapeutic agent and a thermally unstable substance for a sufficient period time to allow for a mass percentage loading of the pores of the porous silicon material(s) with the melted therapeutic agent, or the melted loading mixture comprising the melted therapeutic agent and the melted substance; cooling the infiltrated porous silicon material(s) to allow for solidification of the melted therapeutic agent, or solidification of the melted loading mixture comprising the melted therapeutic agent and the melted substance; and optionally grinding the cooled infiltrated porous silicon material(s) to generate particles that have average diameters less than 100 µm. In a further embodiment, the porous silicon material(s) are porous materials fabricated by a process comprising: electrochemically or stain etching a crystalline silicon containing substrate to generate a porous silicon material; generating porous layers of the silicon material using electropolishing; collecting, drying and fracturing the layers of the porous silicon material to generate porous silicon microparticles; and oxidizing the porous silicon microparticles in air at a temperature of 800 °C or greater for at least 1 hour or greater to generate porous particles. In an alternate embodiment, the porous silicon material(s) are porous silicon materials fabricated by a process comprising: electrochemically or stain etching a crystalline silicon containing substrate to generate a porous silicon material; generating porous layers of the silicon material using electropolishing; collecting, drying and fracturing the layers of the porous silicon material to generate porous silicon microparticles; and oxidizing the porous silicon microparticles in air at a temperature from 25 °C to 700 °C for 1 hour or less to generate porous particles. In another embodiment, the porous silicon material(s) disclosed herein are loaded with 20% to 90% wt/wt of the melted therapeutic agent or the melted loading mixture comprising the melted therapeutic agent and the melted substance. In yet another embodiment, the porous silicon material(s) disclosed herein are loaded with 50% to 75% wt/wt of the melted therapeutic agent or the melted loading mixture comprising the melted therapeutic agent and the melted substance. In a further embodiment, the porous silicon material(s) disclosed herein have a porosity from about 50% to about 80%. In yet a further embodiment, the porous silicon material(s) disclosed herein have a porosity of about 75%. In a certain embodiment, the porous silicon material(s) disclosed herein comprise pores that have diameters from about 2 nm to about 250 nm. In another embodiment, the porous silicon material(s) disclosed herein comprise pores that have diameters from about 20 nm to about 150 nm. In yet another embodiment, the porous silicon material(s) disclosed herein are particles. In a further embodiment, the porous silicon particles disclosed herein have an average dimeter or length from about 10 nm to about 100 µm. In yet a further embodiment, the porous silicon particles disclosed herein have an average dimeter or length from about 10 nm to about 100 nm. In another embodiment, the porous silicon particles disclosed herein have an average dimeter or length from about 100 nm to about 100 µm. In yet another embodiment, the thermally unstable therapeutic agent and the thermally unstable substance are not the same compound or molecule, and wherein the mixture has a lower melting point than the melting point of the thermally unstable therapeutic agent. In a further embodiment, the difference in temperature from which the thermally unstable therapeutic agent begins to melt to when the thermally unstable therapeutic agent begins to degrade is less than about 10 °C. In yet a further embodiment, the difference in temperature from which the thermally unstable therapeutic agent begins to melt to when the thermally unstable therapeutic agent begins to degrade is less than about 5 °C. In another embodiment, the difference in temperature from which the thermally unstable therapeutic agent begins to melt to when the thermally unstable therapeutic agent begins to degrade is from about 2 °C to about 3 °C. In yet another embodiment, the melting point of the loading mixture is at least 10 °C lower than the melting point of the thermally unstable therapeutic agent. In a further embodiment, the melting point of the loading mixture is at least 20 °C lower than the melting point of the thermally unstable therapeutic agent. In yet a further embodiment, the melting point of the loading mixture is at least 30 °C lower than the melting point of the thermally unstable therapeutic agent. In another embodiment, the loading mixture is a eutectic mixture. In yet another embodiment, the loading mixture has a ratio of thermally unstable therapeutic agent to a thermally unstable substance from 1:10 to 10:1. In a certain embodiment, the loading mixture has a ratio of thermally unstable therapeutic agent to a thermally unstable substance from 1:5 to 5:1. In a further embodiment, the loading mixture has a ratio of thermally unstable therapeutic agent to a thermally unstable substance from 1:3 to 3:1. In yet a further embodiment, the thermally unstable substance has a structure that is highly similar to the thermally unstable therapeutic agent. According to the invention, the thermally unstable therapeutic agent is a contraceptive agent. In another embodiment, the contraceptive agent is a progestin or progesterone. In a further embodiment, the thermally unstable substance is cholesterol. In yet a further embodiment, the thermally unstable substance is a second thermally unstable therapeutic agent. In another embodiment, the thermally unstable therapeutic agent and second unstable therapeutic agent are of the same drug class. In yet another embodiment, the thermally unstable therapeutic agent and second unstable therapeutic agent are therapeutics for treating the same medical condition or disorder in a subject. In a further embodiment, the medical condition or disorder is selected from heart disease, diabetes, attention deficit hyperactivity disorder (ADHD), schizophrenia, anxiety, pain, bacterial infections, viral infections, protozoan infections, cancer, motion sickness, hypertension, and symptoms associated with post-menopause. In another embodiment, the thermally unstable therapeutic agent is a progestin or progesterone and the second unstable therapeutic agent is estrogen or a second progestin.

In a certain embodiment, the disclosure further provides for a drug delivery composition made by a method of the disclosure. In a further embodiment, the drug delivery composition of the disclosure is capable of releasing the thermally unstable therapeutic agent *in vivo* or *in vitro* in a linear and sustained manner for at least 30 days. In yet a further embodiment, the drug delivery composition of the disclosure is capable of releasing the thermally unstable therapeutic agent *in vivo* or *in vitro* in a linear and sustained manner for at least 60 days. In another embodiment, the drug delivery composition of the disclosure is capable of releasing the thermally unstable therapeutic agent *in vivo* or *in vitro* in a linear and sustained manner for at least 100 days.

In a particular embodiment, the disclosure provides for a pharmaceutical composition comprising a pharmaceutically acceptable carrier and a drug delivery composition of the disclosure. In a further embodiment, the pharmaceutical composition is formulated for parenteral administration. In yet a further embodiment, the pharmaceutical composition is formulated to be administered intravenously, intra muscularly, or subcutaneously.

In a certain embodiment, the disclosure also provides for drug delivery compositions of the disclosure, or a pharmaceutical composition disclosed herein for use in a method of treating a disease or disorder in a subject in need thereof. In a further embodiment, the disease or disorder is selected from the group consisting of heart disease, diabetes, attention deficit hyperactivity disorder (ADHD), schizophrenia, anxiety, pain, bacterial infections, viral infections, protozoan infections, cancer, motion sickness, hypertension, and symptoms associated with post-menopause.

For use in the therapeutic applications described herein, kits and articles of manufacture are also described herein. Such kits can comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers can be formed from a variety of materials such as glass or plastic.

For example, the container(s) can comprise one or more drug delivery formulations described herein. The container(s) can optionally have a sterile access port (for example the container can be an intravenous solution bag or a vial having a stopper pierceable by a hypodermic injection needle). Such kits optionally comprise an identifying description or label or instructions relating to its use in the methods described herein.

A kit will typically comprise one or more additional materials desirable from a commercial and/or user standpoint for use of a formulation as described herein. Non-limiting examples of such materials include buffers, diluents, filters, needles, syringes; carrier, package, container, vial and/or tube labels listing contents and/or instructions for use, and package inserts with instructions for use.

A label can be on or associated with the container. A label can be on a container when letters, numbers or other characters forming the label are attached, molded or etched into the container itself, a label can be associated with a container when it is present within a receptacle or carrier that also holds the container, e.g., as a package insert. A label can be used to indicate that the contents are to be used for a specific therapeutic application. The label can also indicate directions for use of the contents, such as in the methods described herein. These other therapeutic agents may be used, for example, in the amounts indicated in the Physicians' Desk Reference (PDR) or as otherwise determined by one of ordinary skill in the art.

The following examples are intended to illustrate but not limit the disclosure. While they are typical of those that might be used, other procedures known to those skilled in the art may alternatively be used.

### EXAMPLES

### Example 1

**Fabrication of Porous Silica Particles.** Porous silica particles were obtained through the electrochemical etching of electronics grade silicon wafers (boron doped, p-type, ρ<1 mΩ-cm). Intact 4-inch wafers were mounted in a PEEK holder with electrical contact made on the backside of the wafer. The wafer was then electrochemically etched in a solution of hydrofluoric acid (HF) and ethanol (EtOH) with a platinum counter electrode. The current density and HF concentration was varied based on the particle parameters required. Porous layers were removed from the bulk substrate using an electropolishing step in 1:13 HF:EtOH and current density of 5 mA/cm². Porous silicon films were then collected, dried and fractured into microparticles via an ultrasonic bath in EtOH. Particles were then dried under vacuum and fully oxidized to silica via thermal oxidation in air at 800 °C or higher for a period of time of at least 1 hour. See, *e.g.*, PCT International Publication No. WO2009/009563.

**Evaluation of Drugs for Melt Cast Loading.** To obtain a high drug loading and maintain drug crystallinity, a melt casting approach was chosen. For melt casting, it is important that the drug is stable at its melting temperature and does not begin to burn. To test stability, drugs were analyzed via simultaneous thermal analysis (STA) in which the drug weight and heat flux were measured as a function of temperature. These studies reveal the melting point as well as the temperature at which the drug begins to burn (see **FIG. 2****).** All experiments were performed under nitrogen flow. Five progestins were tested for stability during melt casting: levonorgestrel (LNG), medroxyprogesterone acetate (MPA), etonogestrel (ENG), levonorgestrel butanoate (LNG-B) and segesterone acetate (SEG) (which may also be referred to herein as nestorone (NES)). Of the five tested, three progestins (MPA, LNG-B and NES) had > 20°C differences between their melting and degradation temperatures, indicating that they would likely tolerate a melt casting procedure **(Table 1).** The remaining two progestins, LNG and ENG, both had windows that were deemed too small (2 °C and 3 °C, respectively) to melt cast directly without introducing degradation products. It was further found herein, however, that the LNG and ENG could be melt casted into porous silica by use of eutectic mixtures that greatly extended the windows.

**Table 1. Melting and Degradation temperatures of select progestin drugs.**

| Drug Product | Tₘₑₗₜ (°C) [literature] | Tₘₑₗₜ (°C) [observed] | T_{degradeable} (°C) | Window (°C) [T_{degradeable}-Tₘₑₗₜ] |
|---|---|---|---|---|
| segesterone acetate | 179 | 186 | 219 | 33 |
| Etonogestrel | 184 | 197 | 200 | 3 |
| Levonorgestrel | 240 | 245 | 247 | 2 |
| Levonorgestrel Butanoate | | 214 | 230 | 26 |
| Medroxyprogesterone Acetate | 215 | 217 | 239 | 22 |

SEG does not significantly bind to the estrogen or androgen receptors, which give it a comparatively mild side effect profile to the other progestins screened. To prepare a melt casted formulation, particles and segesterone acetate were combined at the desired ratio as a dry mixture and then elevated to 185 °C under an argon atmosphere. Once segesterone acetate was melted, the mixture was held at temperature for 5 minutes prior to removal from heat and allowed to cool to room temperature. Particles were extracted as a powder and ground using a mortar and pestle to reach the desired particle size.

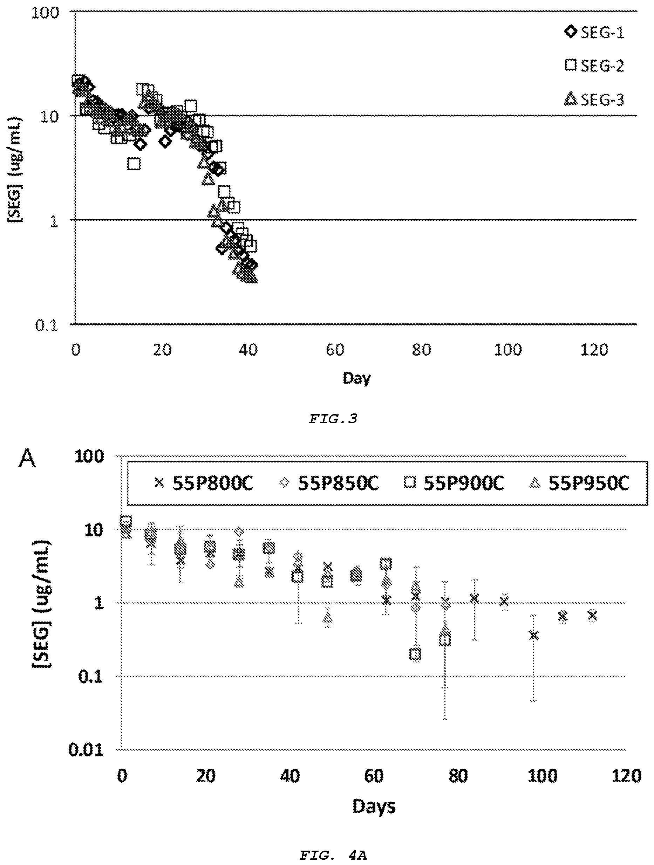

**Modulation of Segesterone Acetate Release Profile.** Three main particle parameters were investigated as to their effect on the release of loaded SEG: particle oxidation temperature, drug loading, and particle porosity. Unless otherwise mentioned, the baseline particle formulation used was 55% porosity, oxidized at 800 °C and had segesterone acetate loading of 70% weight/weight. Drug release was modeled by *in vitro* dissolution in 1X phosphate buffered saline (pH 7.4) under mild agitation at 37 °C. For all studies, starting segesterone acetate concentration was kept constant at 0.4 mg/mL based on the percent loading of the given formulation. Under these conditions, it was found that pure segesterone acetate was fully cleared from the test chamber within 35-40 days, which served as the benchmark for all other formulations (see **FIG. 3****).** Segesterone acetate concentrations were evaluated by HPLC with absorbance measurements at 254 nm.

**Oxidation Temperature & Drug Loading.** Porous silicon particles were oxidized to oxidized porous silicon (which may also be referred to herein as "porous silica") via thermal oxidation in air at temperatures of 800 °C, 850 °C, 900 °C and 950 °C. Without intending to be bound by theory, it is believed that as oxidation temperature increases, the oxide structure relaxes and stabilizes, thus leading to slower dissolution in an aqueous environment. As a trade-off, smaller pores within the system could collapse and become inaccessible to the molten drug during loading. For all oxidation temperatures tested, it was found that processing temperatures in the range 800 °C to 950 °C exerted a minimal influence on the temporal drug release profile (see, e.g., **FIG. 4A****).**

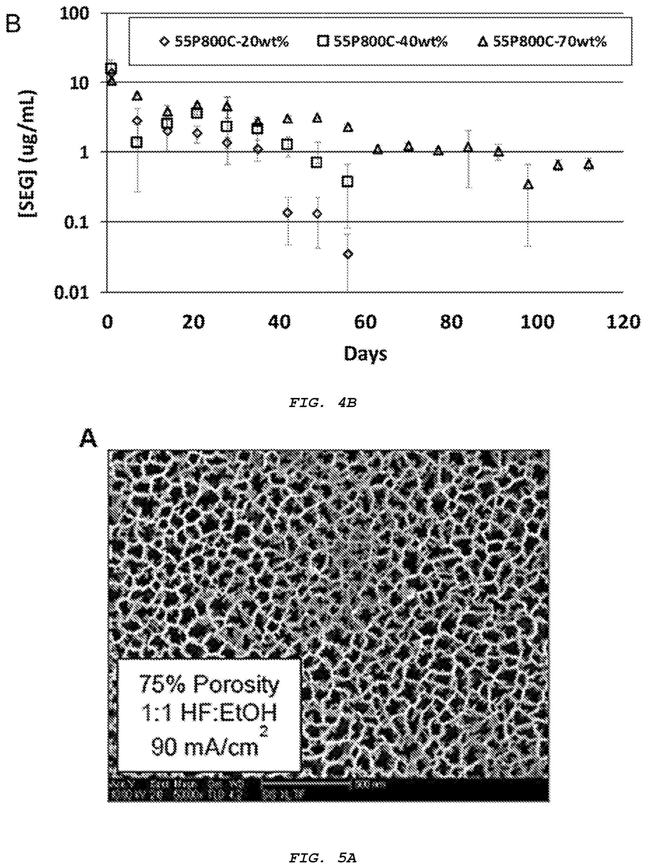

The mass loading of segesterone acetate was also investigated on particle dissolution rate, with weight ratios of 20%, 40% and 70% segesterone acetate tested (see **FIG. 4B****).** By modulating the ratio of drug-to-host, it can change the relative hydrophobic character of the particle surface, while also changing the dosing and duration of action. It was found that by increasing the drug loading, the duration of action could be greatly extended, going from approximately 60 days of segesterone acetate release with 20% segesterone acetate loading to 120 days for a 70% segesterone acetate loading. Interestingly, there was minimal effect on the segesterone acetate concentration detected in the linear portion of the release profile. It should be noted that even the shortest duration of action for SEG-loaded particles exceeded the duration of action for pure segesterone acetate under the same test conditions.

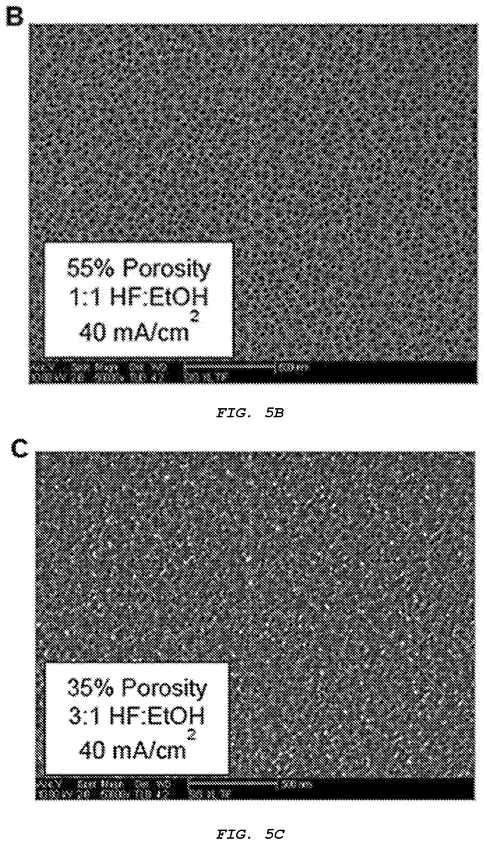

**Pore Morphology.** Of the three parameters investigated, pore morphology had the largest impact on segesterone acetate release in these experiments. Pore morphology is primarily dictated by electrochemical etching parameters, such as current density and HF concentration. For simplicity, the three formulations tested were referenced by their overall particle porosity - 35% (low), 55% (medium) or 75% (high) (see **FIG. 5A-C**).

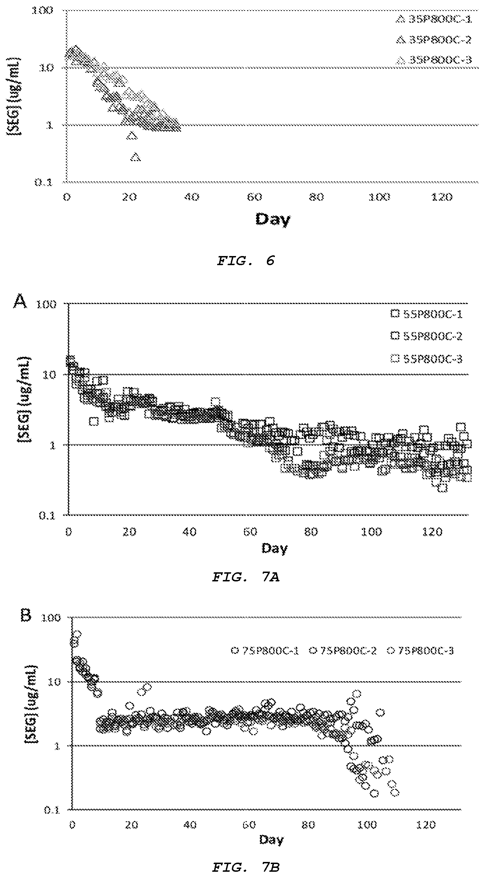

In addition to having more void space, a higher porosity particle also has a larger pore diameter and lower particle density. Increasing the porosity, along with modifying the surface area and morphology of the particle, also improves drug penetration. For low porosity particles, the majority of the drug is understood to have remained on the outside of the particle, with pore volume decreasing less than 5% after melt casting procedures (measured by N₂ adsorption, density functional theory model). The poor drug penetration leads to a release curve that looks very similar to the pure drug control, with no detectable segesterone acetate released after day 40 (see **FIG. 6****).**

For the two higher porosity samples tested, 55% and 75% porosity, the pore openings are understood to be sufficiently large to allow for the molten drug to enter the pores. After melt infiltration, medium porosity samples lost approximately 45% of their available pore volume while high porosity samples lost 55%. Incorporating the melted drug into the porous matrix allowed for a significantly extended release, and for higher porosity samples, a highly linear release with a rapid drop-off (see **FIG. 7****).**

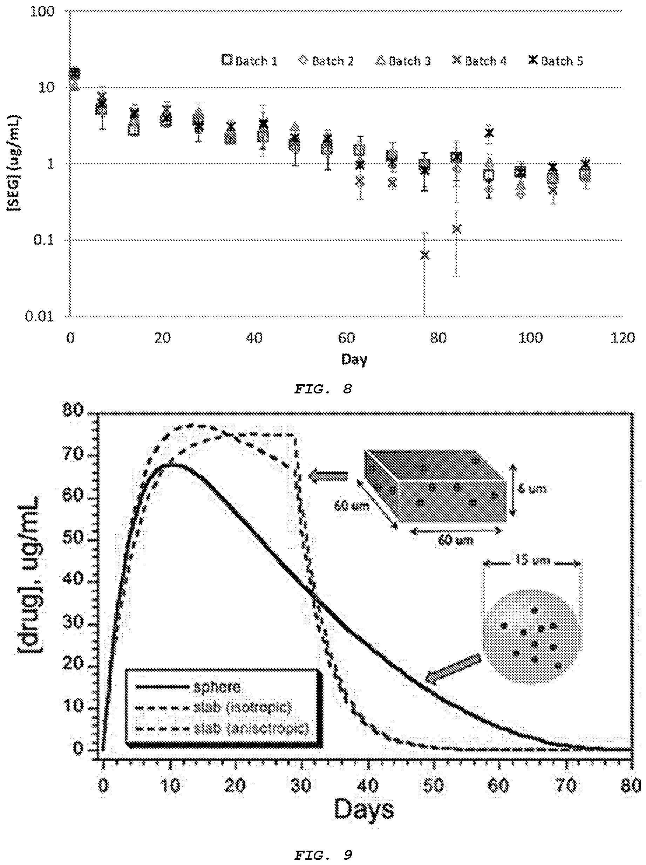

**Batch-to-Batch Variability.** An additional set of studies were performed on the batch-to-batch variation of melt casted particles. For this study, 55% porosity, 800°C oxidation and 70% weight loading were used as the baseline formulation. For each unique batch, a separate silicon wafer was used as the starting material and then etched, ultrasonicated, dried, oxidized and melt infiltrated separately from the other batches. Mass loading for five unique batches was determined by thermogravimetric analysis to be 70.6% with a standard deviation of 2.3%. For the five batches tested, their release profiles were tightly distributed, indicating a high level of reproducibility (see **FIG. 8****).**

***In Vivo* Performance.** *In vivo* studies were performed in female adult Sprague-Dawley rats, weighing approximately 300 g each. Two studies were conducted, a short-term tolerability test followed by a longer-term study. Prior to the start of the study, particles were screened by a third-party facility (Nelson Labs) and came back negative for aerobic bacteria or fungal colonies. Animals received a subcutaneous injection in the scruff of the neck containing 50 mg/kg SEG. For short term studies, empty porous silica particles as well as medium and high porosity 70wt% segesterone acetate loaded particles were administered. After three weeks, animals were sacrificed and the brain, heart, lungs, kidney, spleen and liver were collected, fixed, sectioned and stained with hematoxylin and eosin (H&E). A trained pathologist performed a blind exam of the tissue sections and found no obvious signs of toxicity. Some background lesions were noted, such as mild vacuolar hepatopathy in the liver and microuroliths in the kidneys, but they occurred at approximately the same rate in the dosed animals as in the control.

For longer term studies, five experimental groups (n = 5 animals each) were studied: empty porous silica particles, pure non-micronized SEG, medium porosity 70 wt% segesterone acetate loaded particles and two groups of two different batches of high porosity 70 wt% segesterone acetate loaded particles. The five groups were monitored for 6 months with body weight measurements and tail-vein blood draws taken weekly. All SEG-containing formulations saw an increase in body weight initially, but returned to baseline levels after 8 weeks. No animals showed overt signs of segesterone acetate or particle-induced toxicity throughout the study. Two rats, one empty particle control and one pure segesterone acetate control, developed mammary tumors during study and were sacrificed prior to the 6-month mark, but histopathology revealed benign fibroadenomas which are unfortunately common in female research rats. It was deemed unlikely that the particles or segesterone acetate were the cause of these tumors.

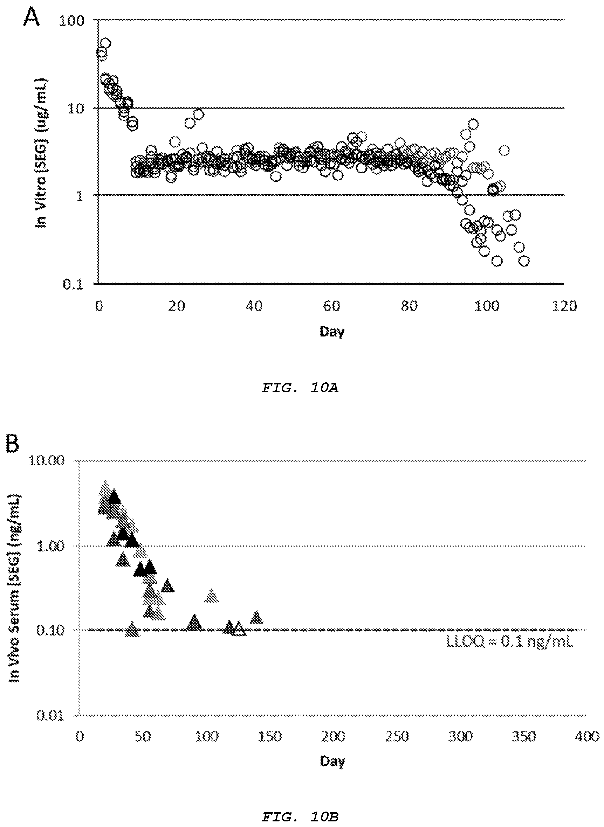

Serum samples were processed and submitted to a third-party lab for segesterone acetate detection via HPLC-MS/MS. Although the majority of samples submitted were below the limit of quantification for this assay (LLOQ = 0.1 ng/mL), serum concentrations were obtained for the earliest time points (see **FIG. 10****).** For high porosity samples, a similar shape of the release curve was observed, but over a much larger time scale. For *in vitro* testing, the burst regime of 75% porosity particles was only observed for 8 days, but *in vivo* can be seen up to 8 weeks. segesterone acetate concentrations for terminal, 6-month timepoints were analyzed using an internal HPLC-MS/MS and segesterone acetate was still detected in the non-micronized, pure segesterone acetate control as well as in the 75% porosity dosed animals. These results clearly demonstrate that the *in vitro* model used to predict dissolution rates was much more aggressive than the conditions in the subcutaneous space of the rates, leading to dissolution rates at least 6 times faster than those observed *in vivo.* Using the approximate shape of the serum levels compared to the *in vitro* model, it is predicted that a 75% porosity, 70wt% segesterone acetate loaded formulation could still be releasing segesterone acetate in the linear regime up to 24 months post-injection.

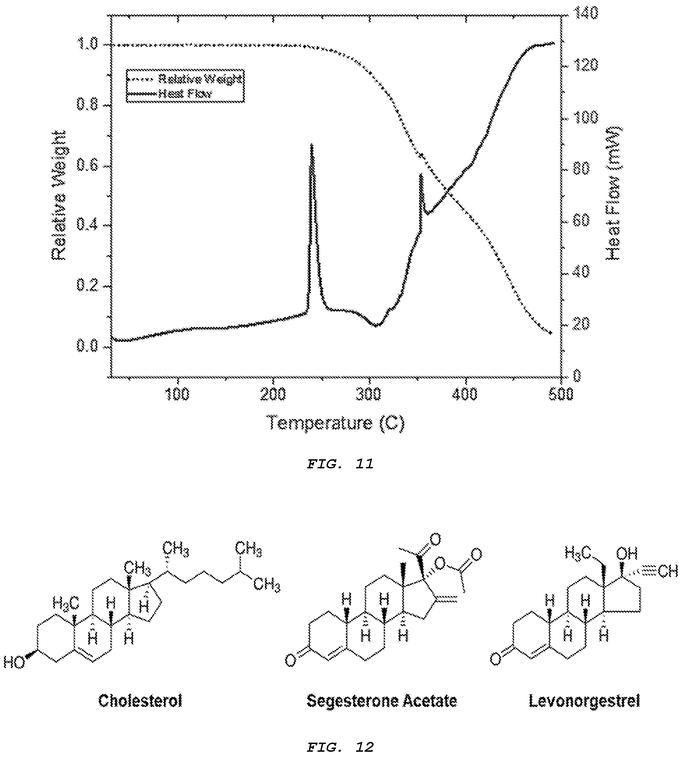

**Loading of Thermally Unstable Compounds into Porous Silica Particles by Melt Infiltration.** To expand the catalogue of drugs available for delivering from porous silicon (Psi) hosts, levonorgestrel (LNG) was evaluated. LNG is most commonly used as the active ingredient in emergency oral contraceptive methods ("Plan B"), but it is also available in daily oral birth control pills (such as Norgeston and Levora), intrauterine devices (Mirena) and long-term implants (Norplant). Prior to melt casting, LNG was screened for thermal stability (see **FIG. 11****).** It was found that LNG began to melt at approximately 233 °C (literature value: 238 °C) but began to lose mass at the same temperature, indicating degradation and making it an unsuitable candidate for melt casting in porous silica materials without modification of the technique.

To depress the melting point of LNG below its degradation temperature, a strategy of adding a melting point suppression agent was employed. Without intending to be bound by theory, the introduction of the additional agent to the composition is understood to weaken the intermolecular bonds in the LNG lattice, thus leading to reduced energy required to induce melting. Two test molecules were chosen as the melting point suppression agent: cholesterol (CHOL) and segesterone acetate (SEG) (see **FIG. 12****).** All three molecules, LNG, CHOL and SEG, belong to the broad steroid family of molecules (see above) and share similar structural motifs, including three cyclohexane rings fused with a cyclopentane ring.

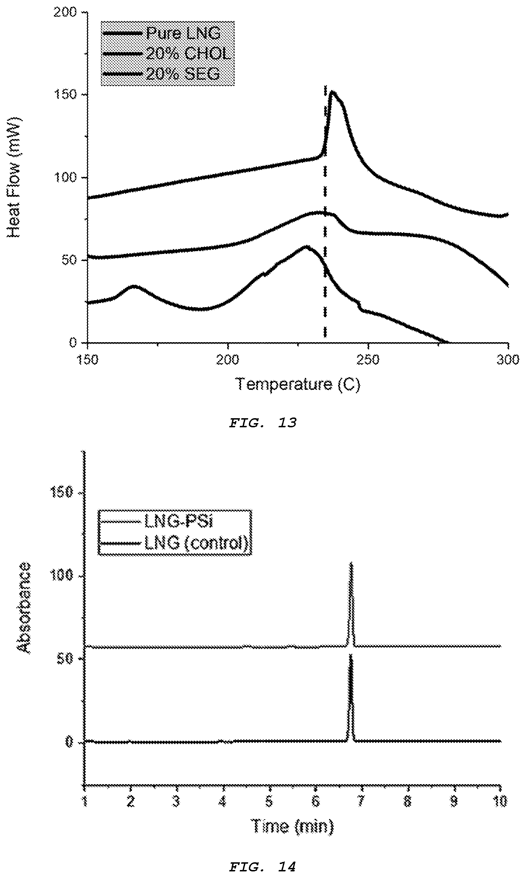

SEG and CHOL were chosen as melting point suppression agents due to their structural similarities to LNG likely having the largest effect on melting point, but the phenomenon of melting point depression is not limited to just molecules from the same family. LNG was mechanically mixed with 20% (wt/wt) of either segesterone acetate or CHOL and then heated under O₂ atmosphere to evaluate the melting point of the mixture (see **FIG. 13****).** It was found that both segesterone acetate and CHOL caused significant broadening and overall depression of the melting point of LNG. For LNG mixed with segesterone acetate or CHOL, endothermic behavior associated with the onset of melting was observed to begin starting at 195 °C and 205 °C, respectively. Both segesterone acetate and CHOL were deemed to be suitable for depressing the melting point of LNG sufficiently to enable its melt casting into oxidized porous silicon materials without inducing thermal degradation.

A mixture of 80% LNG and 20% CHOL was then prepared and mixed in a 2:1 ratio with oxidized porous silicon microparticles. Oxidized porous silicon microparticles were prepared using a similar method to those used for segesterone acetate melt casting described elsewhere (75% porosity, 800 °C oxidation). The mixture of oxidized porous silicon, LNG and CHOL was mechanically mixed and raised to a temperature of 215 °C in a nitrogen atmosphere. Once molten, the LNG-CHOL mixture was left to infiltrate the porous silica materials for 5 minutes before cooling to room temperature. The oxidized porous silicon material containing LNG-CHOL was then recovered and ground into microparticles using a mortar and pestle. The loaded oxidized porous silicon particles were then evaluated for their release kinetics in 1X PBS (pH 7.4) at 37 °C. Approximately 2 mg of LNG-PSi were placed in a glass vial containing 3.5 mL of 1X PBS and placed in a 37 °C incubator on a moving platform to induce mild agitation of the solution. 3 mL of the PBS was removed every 24 hours and replaced with fresh solution. The extracted supernatant was then analyzed for LNG content by high performance liquid chromatography (HPLC). It was found that LNG released from the loaded oxidized porous silicon particles, when analyzed by HPLC, retained the same peak shape, elution time and number of peaks as pure LNG standards (see **FIG.14****).** These results demonstrate that LNG is not chemically altered or degraded by the melt casting process.

Release kinetics of LNG from PSi after melt casting was then evaluated over a 6-month period (see **FIG. 15****).** It was found that LNG release was relatively sustained and linear over the 6-month period (ranging from 1 ug/mL to 0.1 ug/mL).

**Loading of a Thermally Unstable Antibiotic into Oxidized Porous Silicon Particles by Melt Infiltration** (not according to the invention). In addition to contraceptive hormones, additional families of drugs have been investigated for their viability for melt casting. One such drug included rifampin (RFP), a rifamycin family molecule used as a potent antibiotic. Similar to levonorgestrel, pure RFP would be deemed unsuitable for melt casting due to a narrow window of thermal stability. Specifically, it was found that RFP began melting at approximately 178 °C but began losing mass from volatile degradation products at 184 °C. To broaden the window, a similarly structured molecule, rapamycin (RAPA), was mechanically mixed with RFP at a ratio of 4:1 (RFP:RAPA) (see **FIG. 16****).** The mixture comprising RFP and RAPA was then evaluated for its ability to be melt cast. These experiments demonstrated that the incorporation of RAPA significantly depressed and broadened the temperature range of RFP melting, thus lowering and broadening the window of thermal stability for melt casting (see **FIG. 17****).**

### Example 2

**Materials and Methods.** Melt-casted samples were prepared by mixing dry porous silica microparticles (75% porosity, 800°C oxidation) with a dry mixture containing 80% levonorgestrel (Industriale Chimica, USP grade) and 20% cholesterol (Sigma-Aldrich, >92.5% purity from sheep wool) by mass. Samples were purged thoroughly with argon and heated to 235°C under positive argon pressure for 5 minutes. Samples were allowed to cool to room temperature before processing in a mortar and pestle. Diffractograms were collected from 2θ = 15-40° using Cu kα (1.54Å) radiation, a step size of 0.026° and a scan time of 1s per step.

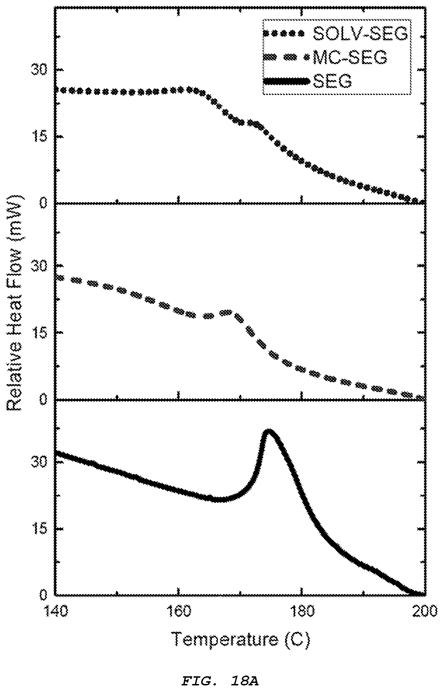

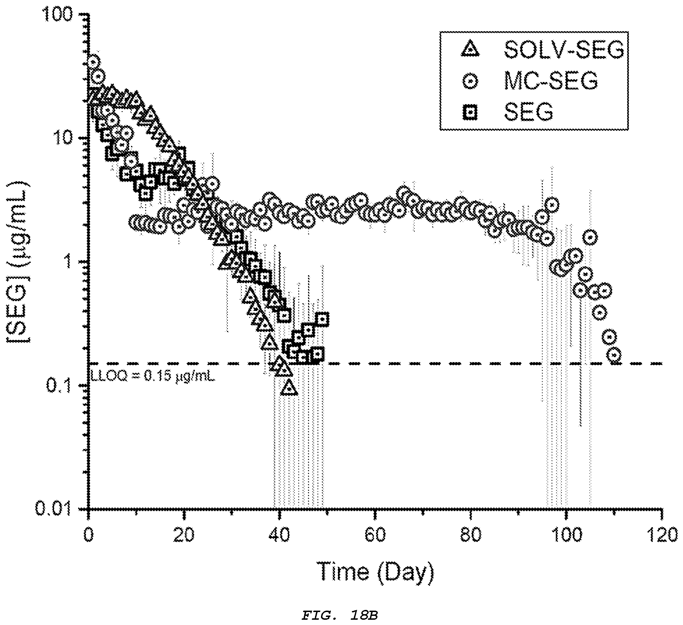

In previous work with segesterone acetate (SEG, also known as Nestorone), crystallinity was a predictor of sustained release. It was found that samples loaded with SEG via solvent evaporation had minimal melting events relative to those loaded via melt casting **(****Fig. 18A****),** which is a sign of reduced crystallinity. By reducing overall crystallinity of SEG, dissolution kinetics were accelerated and there was no discernable benefit of loading SEG into porous silica microparticles by solvent evaporation **(****FIG. 18B****).**

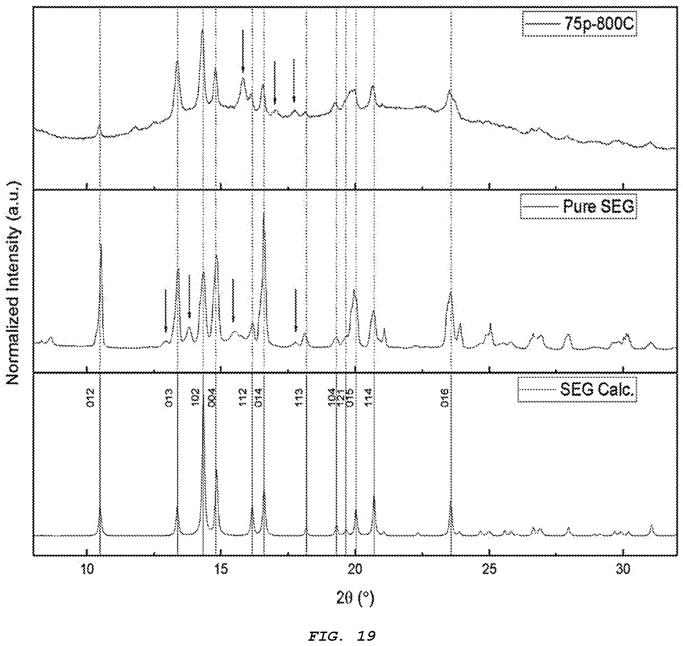

Upon further evaluation of SEG crystallinity within porous silicon (PSi), there were several features of note found within the diffractograms of SEG loaded into PSi **(****FIG. 19****).** The first is the presence of additional peaks not found within the theoretical pattern for pure SEG, likely due to a polymorphism from the manufacturing process or prolonged storage. These additional peaks, marked by arrows (middle panel), were not found after melt casting which would confirm that SEG fully melted and re-crystallized during the melt casting process. However, new peaks (marked with arrows top panel) also appeared, indicating that perhaps new polymorphisms arise during melt casting.

In addition, the relative peak intensities of several characteristic SEG peaks were altered during the melt casting process. This is most apparent in the suppression of the (012) and (014) planes and activation of the (102) plane. These results suggest a preferred orientation of the SEG crystal when reformed within the pore of the PSi particles.

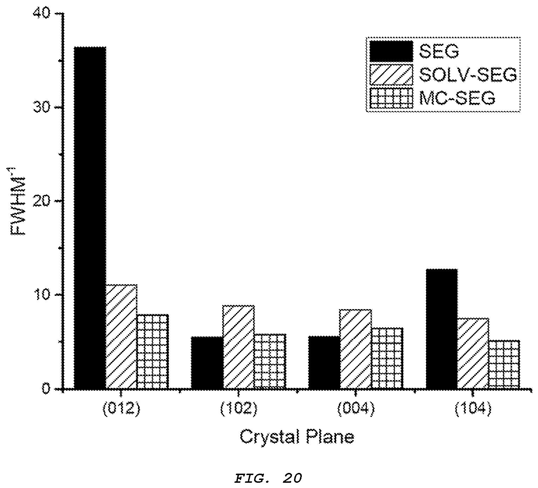

In addition, the full width half max (FWHM) of x-ray diffraction peaks is inversely proportional to crystallite size. As seen in **FIG. 20****,** the FWHM was evaluated for solvent evaporation (SOLV-SEG) and melt casted SEG formulations. Both SOLV-SEG and MC-SEG formulations had similar crystallite size distributions, which differed greatly from the pure SEG samples. Again, this suggests full recrystallization within the pores of PSi. Interestingly, SOLV-SEG particles had crystallite sizes consistently larger than MC-SEG, but at an overall much lower level of crystallinity. For evaluating PSi formulations with LNG-CHOL mixtures, a similar approach was taken. Compared to the pure LNG crystals, a clear suppression of peaks at 2θ = 17° and 22.5° are observed **(****FIG. 20****),** also suggesting a full melting and re-crystallization within the pores of PSi, resulting in a preferred orientation.

## Claims

1. A drug delivery formulation comprising:
a porous silicon material loaded with a meltable composition,
wherein the meltable composition comprises a therapeutic agent and a melting point suppression agent, wherein the therapeutic agent is a contraceptive drug selected from the group consisting of segesterone, etonogestrel, levonorgestrel, levonorgestrel butanoate, and medroxyprogesterone acetate, and wherein the melting point suppression agent is a steroid or a polyketide,
wherein the meltable composition has a melting temperature,
wherein the therapeutic agent has a melting temperature and a decomposition temperature, and
wherein the melting temperature of the meltable composition is lower than the melting temperature of the therapeutic agent and the decomposition temperature of the therapeutic agent.

2. The formulation of claim 1, wherein the formulation is prepared by melt casting the composition into the porous silicon material at a temperature above the melting temperature of the composition and below the decomposition temperature of the therapeutic agent.

3. The formulation of claim 1, wherein the meltable composition is a eutectic mixture; preferably,
wherein the eutectic mixture has a eutectic temperature that is below the decomposition temperature of the therapeutic agent.

4. The formulation of claim 1, wherein the melting temperature of the therapeutic agent is no more than 50 °C, 20 °C, 10 °C, 5 °C, 2 °C, or 1 °C lower than the decomposition temperature of the therapeutic agent; and/or,
wherein the melting temperature of the meltable composition is at least 1 °C, 2 °C, 5 °C, 10 °C, 20 °C, or 50 °C lower than the decomposition temperature of the therapeutic agent.

5. The formulation of claim 1, wherein the melting point suppression agent is (i) cholesterol or
(ii) rapamycin.

6. The formulation of claim 1, wherein the melting point suppression agent is a steroid; preferably,
wherein the therapeutic agent is levonorgestrel, and the melting point suppression agent is cholesterol.

7. The formulation of claim 1, wherein the porous silicon material is loaded to at least 20%, at least 40%, or at least 70% weight/weight with the meltable composition; or,
wherein the porous silicon material has a porosity of at least 35%, at least 55%, at least 75%, or from 15% to 85%.

8. The formulation of claim 1, wherein the formulation releases the therapeutic agent into an aqueous solution more slowly than the therapeutic agent is released from a formulation comprising the porous silicon material loaded with the therapeutic agent without the melting point suppression agent.

9. The formulation of claim 1, wherein the porous silicon material is an oxidized porous silicon material; preferably,
wherein the porous silicon material has been oxidized at a temperature of 800 °C or greater for 1 hour or longer.

10. The formulation of claim 1, wherein the porous silicon material is a particulate material; preferably,
wherein the particulate material has an average diameter or length of from 10 nm to 100 µm.

11. A pharmaceutical composition comprising the drug delivery formulation of any one of claims 1-10 and a pharmaceutically acceptable carrier.

12. The drug delivery formulation according to any one of claims 1-10 or the pharmaceutical composition according to claim 11, for use in preventing pregnancy in a human female subject in need thereof; preferably,
wherein the human female subject is in need of contraception; optionally,
wherein the drug delivery formulation or pharmaceutical composition is for parenteral administration; preferably,
wherein the drug delivery formulation or pharmaceutical composition is for subcutaneous administration.

13. The drug delivery formulation or pharmaceutical composition for use according to claim 12, wherein the therapeutic agent of the drug delivery formulation or pharmaceutical composition is released in the human female subject for an extended time period; preferably, wherein the therapeutic agent is released in the human female subject for at least 60 days.

## Patentansprüche

1. Wirkstofffreisetzungsformulierung, umfassend:
ein poröses Siliziummaterial, das mit einer schmelzbaren Zusammensetzung geladen ist, wobei die schmelzbare Zusammensetzung ein Therapeutikum und ein Schmelzpunktsenkungsmittel umfasst, wobei das Therapeutikum ein Verhütungsmittel ist, das aus der Gruppe ausgewählt ist, die aus Segesteron, Etonogestrel, Levonorgestrel, Levonorgestrelbutanoat und Medroxyprogesteronacetat besteht, und wobei das Schmelzpunktsenkungsmittel ein Steroid oder ein Polyketid ist,
wobei die schmelzbare Zusammensetzung eine Schmelztemperatur aufweist,
wobei das Therapeutikum eine Schmelztemperatur und eine Zersetzungstemperatur aufweist, und
wobei die Schmelztemperatur der schmelzbaren Zusammensetzung niedriger ist als die Schmelztemperatur des Therapeutikums und die Zersetzungstemperatur des Therapeutikums.

2. Formulierung nach Anspruch 1, wobei die Formulierung durch Schmelzgießen der Zusammensetzung in das poröse Siliziummaterial bei einer Temperatur über der Schmelztemperatur der Zusammensetzung und unter der Zersetzungstemperatur des Therapeutikums hergestellt wird.

3. Formulierung nach Anspruch 1, wobei die schmelzbare Zusammensetzung ein eutektisches Gemisch ist; vorzugsweise,
wobei das eutektische Gemisch eine eutektische Temperatur aufweist, die unter der Zersetzungstemperatur des Therapeutikums liegt.

4. Formulierung nach Anspruch 1, wobei die Schmelztemperatur des Therapeutikums nicht mehr als 50 °C, 20 °C, 10 °C, 5 °C, 2 °C oder 1 °C niedriger ist als die Zersetzungstemperatur des Therapeutikums; und/oder
wobei die Schmelztemperatur der schmelzbaren Zusammensetzung mindestens 1 °C, 2 °C, 5 °C, 10 °C, 20 °C oder 50 °C niedriger ist als die Zersetzungstemperatur des Therapeutikums.

5. Formulierung nach Anspruch 1, wobei das Schmelzpunktsenkungsmittel (i) Cholesterin ist;
oder
(ii) Rapamycin.

6. Formulierung nach Anspruch 1, wobei das Schmelzpunktsenkungsmittel ein Steroid ist; vorzugsweise,
wobei das Therapeutikum Levonorgestrel ist und das Schmelzpunktsenkungsmittel Cholesterin ist.

7. Formulierung nach Anspruch 1, wobei das poröse Siliziummaterial auf mindestens 20 %, mindestens 40 % oder mindestens 70 % Gewicht/Gewicht mit der schmelzbaren Zusammensetzung geladen wird; oder
wobei das poröse Siliziummaterial eine Porosität von mindestens 35 %, mindestens 55 %, mindestens 75 % oder von 15 % bis 85 % aufweist.

8. Formulierung nach Anspruch 1, wobei die Formulierung das Therapeutikum in eine wässrige Lösung langsamer freisetzt, als das Therapeutikum aus einer Formulierung freigesetzt wird, die das mit dem Therapeutikum ohne das Schmelzpunktsenkungsmittel geladene poröse Siliziummaterial umfasst.

9. Formulierung nach Anspruch 1, wobei das poröse Siliziummaterial ein oxidiertes poröses Siliziummaterial ist; vorzugsweise,
wobei das poröse Siliziummaterial bei einer Temperatur von 800 °C oder höher für 1 Stunde oder länger oxidiert wurde.

10. Formulierung nach Anspruch 1, wobei das poröse Siliziummaterial ein teilchenförmiges Material ist; vorzugsweise
wobei das teilchenförmige Material einen mittleren Durchmesser oder eine Länge von 10 nm bis 100 µm aufweist.

11. Pharmazeutische Zusammensetzung, die die Wirkstofffreisetzungsformulierung nach einem der Ansprüche 1-10 und einen pharmazeutisch annehmbaren Träger umfasst.

12. Wirkstofffreisetzungsformulierung nach einem der Ansprüche 1-10 oder pharmazeutische Zusammensetzung nach Anspruch 11, zur Verwendung bei der Verhinderung einer Schwangerschaft bei einer menschlichen weiblichen Person, die jener bedarf, vorzugsweise,
wobei die menschliche weibliche Person einer Verhütung bedarf; wahlweise,
wobei die Wirkstofffreisetzungsformulierung oder pharmazeutische Zusammensetzung zur parenteralen Verabreichung ist; vorzugsweise,
wobei die Wirkstofffreisetzungsformulierung oder pharmazeutische Zusammensetzung zur subkutanen Verabreichung ist.

13. Wirkstofffreisetzungsformulierung oder pharmazeutische Zusammensetzung zur Verwendung nach Anspruch 12, wobei das Therapeutikum der Wirkstofffreisetzungsformulierung oder pharmazeutischen Zusammensetzung in der menschlichen weiblichen Person über einen längeren Zeitraum freigesetzt wird; wobei das Therapeutikum in der menschlichen weiblichen Person vorzugsweise über mindestens 60 Tage freigesetzt wird.

## Revendications

1. Formulation d'administration de médicament comprenant :
un matériau de silicium poreux chargé d'une composition fusible,
dans laquelle la composition fusible comprend un agent thérapeutique et un agent de suppression de point de fusion, dans laquelle l'agent thérapeutique est un médicament contraceptif sélectionné parmi le groupe consistant en ségestérone, étonogestrel, lévonorgestrel, butanoate de lévonorgestrel, et acétate de médroxyprogestérone, et dans laquelle l'agent de suppression de point de fusion est un stéroïde ou un polykétide,
dans laquelle la composition fusible présente une température de fusion,
dans laquelle l'agent thérapeutique présente une température de fusion et une température de décomposition, et
dans laquelle la température de fusion de la composition fusible est inférieure à la température de fusion de l'agent thérapeutique et à la température de décomposition de l'agent thérapeutique.

2. Formulation selon la revendication 1, dans laquelle la formulation est préparée par coulée par fusion de la composition dans le matériau de silicium poreux à une température supérieure à la température de fusion de la composition et inférieure à la température de décomposition de l'agent thérapeutique.

3. Formulation selon la revendication 1, dans laquelle la composition fusible est un mélange eutectique ; préférablement,
dans laquelle le mélange eutectique présente une température eutectique qui est inférieure à la température de décomposition de l'agent thérapeutique.

4. Formulation selon la revendication 1, dans laquelle la température de fusion de l'agent thérapeutique n'est pas supérieure à 50 °C, 20 °C, 10 °C, 5 °C, 2 °C, ou 1 °C inférieure à la température de décomposition de l'agent thérapeutique ; et/ou,
dans laquelle la température de fusion de la composition fusible est au moins 1 °C, 2 °C, 5 °C, 10 °C, 20 °C, ou 50 °C inférieure à la température de décomposition de l'agent thérapeutique.

5. Formulation selon la revendication 1, dans laquelle l'agent de suppression de point de fusion est (i) du cholestérol ;
ou
(ii) de la rapamycine.

6. Formulation selon la revendication 1, dans laquelle l'agent de suppression de point de fusion est un stéroïde ; préférablement,
dans laquelle l'agent thérapeutique est du lévonorgestrel, et l'agent de suppression de point de fusion est du cholestérol.

7. Formulation selon la revendication 1, dans laquelle le matériau de silicium poreux est chargé à au moins 20 %, au moins 40 %, ou au moins 70 % en poids/poids de la composition fusible ; ou,
dans laquelle le matériau de silicium poreux présente une porosité d'au moins 35 %, d'au moins 55 %, d'au moins 75 %, ou de 15 % à 85 %.

8. Formulation selon la revendication 1, dans laquelle la formulation libère l'agent thérapeutique dans une solution aqueuse plus lentement que l'agent thérapeutique est libéré à partir d'une formulation comprenant le matériau de silicium poreux chargé de l'agent thérapeutique sans l'agent de suppression de point de fusion.

9. Formulation selon la revendication 1, dans laquelle le matériau de silicium poreux est un matériau de silicium poreux oxydé ; préférablement,
dans laquelle le matériau de silicium poreux a été oxydé à une température de 800 °C ou supérieure pendant 1 heure ou plus.

10. Formulation selon la revendication 1, dans laquelle le matériau de silicium poreux est un matériau particulaire ; préférablement,
dans laquelle le matériau particulaire présente un diamètre moyen ou une longueur moyenne de 10 nm à 100 µm.

11. Composition pharmaceutique comprenant la formulation d'administration de médicament selon l'une quelconque des revendications 1 à 10 et un vecteur pharmaceutiquement acceptable.

12. Formulation d'administration de médicament selon l'une quelconque des revendications 1 à 10 ou la composition pharmaceutique selon la revendication 11, pour une utilisation dans la prévention d'une grossesse chez un sujet humain de sexe féminin en ayant besoin ; préférablement,
dans laquelle le sujet humain de sexe féminin a besoin d'une contraception ; facultativement,
dans laquelle la formulation d'administration de médicament ou la composition pharmaceutique est destinée à une administration parentérale ; préférablement,
dans laquelle la formulation d'administration de médicament ou la composition pharmaceutique est destinée à une administration sous-cutanée.

13. Formulation d'administration de médicament ou composition pharmaceutique destinée à une utilisation selon la revendication 12, dans laquelle l'agent thérapeutique de la formulation d'administration de médicament ou de la composition pharmaceutique est libéré chez le sujet humain de sexe féminin pendant une période de temps prolongée ; préférablement, dans laquelle l'agent thérapeutique est libéré chez le sujet humain de sexe féminin pendant au moins 60 jours.
